# EUROPEAN PATENT SPECIFICATION

(11) **EP 1 969 369 B1**
(45) Date of publication and mention of the grant of the patent: **01.08.2012**
(21) Application number: 06843122.0
(22) Date of filing: 19.12.2006
(51) Int. Cl.: G01N 33/68

(54) **NOVEL CAPTURE AGENTS FOR BINDING A LIGAND**
NEUE EINFANGMITTEL ZUR BINDUNG EINES LIGANDEN
NOUVEAUX AGENTS DE CAPTURE POUR LA LIAISON À UN LIGAND

(30) Priority: 20.12.2005 GB 0525915
(43) Date of publication of application: 17.09.2008
(60) Divisional application: 11187319.6
(73) Proprietor: Sharp Kabushiki Kaisha, Osaka-shi, Osaka 545-8522 (JP)
(72) Inventor: REEVE, Michael, A., Henley-on-Thames, Oxfordshire RG9 1PL (GB); ANDERSON, Sally, Oxford OX2 6RQ (GB)
(74) Representative: Baldock, Sharon Claire
(86) International application number: PCT/JP2006/325695
(87) International publication number: WO 2007/072973

(56) References cited:
- EP-A- 1 969 371
- WO-A-02/064619
- WO-A1-00/52456
- WO-A2-2004/058946
- JP-A- 03 083 600
- JP-A- 2000 510 233
- JP-A- 2005 504 309
- JP-A- 2005 516 595
- JP-A- 2005 524 057
- GIBNEY B R ET AL: "Hydrophobic modulation of heme properties in heme protein maquettes", BIOCHEMISTRY, AMERICAN CHEMICAL SOCIETY, US, vol. 40, no. 35, 4 September 2001 (2001-09-04), pages 10550-10561, XP009142346, ISSN: 0006-2960, DOI: DOI:10.1021/BI002806H [retrieved on 2001-08-11]
- SHELLY J DORMADY ET AL: "Eliminating disulfide exchange during glutamyl endopeptidase digestion of native protein", JOURNAL OF CHROMATOGRAPHY, ELSEVIER SCIENCE PUBLISHERS B.V, NL, vol. 864, 1 January 1999 (1999-01-01), pages 237-245, XP007916432, ISSN: 0021-9673
- KAZUNORI MATSUURA ET AL: "Artificial Peptide-Nanospheres Self-Assembled from Three-Way Junctions of alpha-Sheet-Forming Peptides", JOURNAL OF THE AMERICAN CHEMICAL SOCIETY, AMERICAN CHEMICAL SOCIETY, US, vol. 127, 1 January 2005 (2005-01-01), pages 10148-10149, XP007916431, ISSN: 0002-7863, DOI: DOI:10.1021/JA052644I [retrieved on 2005-06-30]

## Description

### TECHNICAL FIELD

The current invention relates arrays of capture agents for binding ligands. Methods of making these capture agents, as well as methods of identifying capture agents which bind to a specific ligand of interest, are also described

### BACKGROUND ART

Traditional methods for producing libraries of capture agents, such as the production of antibody libraries are both laborious and expensive. Therefore, a number of attempts have been made to synthetically produce libraries of peptides which can act as capture agents to bind various types of ligand.

Spatially addressable libraries and one-bead one-structure libraries have previously been described for use in screening methods. These can be either a ligand library against a specific receptor, or a receptor library against a specific ligand (Combinatorial Chemistry and high throughput screening, 1, 113, 1998).

Pro. SPIE, 4205, 75 (2001), describes the use of cyclohexapeptides bound to quartz surfaces derivatised with epoxides, or directly to gold surfaces. This document describes peptides in which every other amino acid is varied. The peptides are attached to the surfaces by either lysyl or cysteiyl residues. Binding of amino acids to the surface bound peptides is then assayed.

Cyclic pentapeptide libraries for use in the identification of enzyme inhibitors have also been previously disclosed. Libraries of cyclic and linear peptides were made and iterative synthesis and screening was used to generate enzyme inhibitors (Molecular Diversity, 1, 223, 1995).

WO2005/047502 also discloses a protein library consisting of proteins having random amino acid sequences in which 4 to 12 types of amino acid are present, wherein the amino acids comprise Gly, Ala, Val and Glu or Asp. It also discloses a method of screening for a protein having a particular structure or function from the library.

These prior art libraries are able to be produced more rapidly than traditional antibody libraries, but still suffer from the problem that they are expensive to produce and also require a large number of individual peptides to be synthesised in order to provide sufficient sequence diversity to make the libraries effective.

### DISCLOSURE OF INVENTION

It is therefore an object of the current invention to provide arrays of synthetic capture agents having increased sequence diversity.

In accordance with the invention there is provided an array of peptide dimer ligand-binding capture agents for capturing an analyte in a sample wherein:
each of said ligand-binding capture agents comprises first and second peptide chains being formed as a dimer by means of a covalent link therebetween, wherein said covalent link has been formed by reaction of first and second thiol groups on said first and second peptides,
each of said first and second peptide chains comprises a chain of between 5 and 25 amino acids wherein each amino acid is a D-enantiomer or an L-enantiomer and has been selected from a set of
each of said first and second peptide chains have different amino acid sequences,
said first peptide is immobilised via an attachment moiety to a substrate, said attachment moiety being selected from the group consisting of a hydrophobic moiety and a moiety forming a covalent bond with the substrate and wherein
the first and second peptide chains are combinatorially-varied, such that all possible combinations of amino acids present in the said set are present in the array and said array comprises discrete spatially addressable locations and each location comprises a different capture agent.

Preferably, each amino acid has been selected from a set of 4 amino acids.

Preferably, the ligand comprises a eukaryotic cell, a prokaryotic cell, a virus, a bacteriophage, a prion, a spore, a pollen grain, an allergen, a nucleic acid, a protein, a peptide, a carbohydrate, a lipid, an inorganic compound or an organic compound.

There is also described herein a dimeric capture agent for binding a ligand, and for incorporation into the array of the invention said capture agent comprising at least first and second peptide chains, wherein said first and second peptide chains each comprise a chain of between 2 and 25 amino acids, each of said amino acids being substantially enantiomerically pure, and wherein said at least first and second peptide chains are covalently linked.

Preferably, each amino acid is selected from a set consisting essentially of less than 6 amino acids and most preferably 4 amino acids.

It will be understood that each substantially enantiomerically pure amino acid monomer can be an L-amino acid, or a D-amino acid.

Preferably, the first and second peptides are synthesised on a solid phase, more preferably, the peptides are cleaved from the solid phase prior to use in the first aspect.

Syntheses of peptides and their salts and derivatives, including both solid phase and solution phase peptide syntheses, are well established in the art. See, e.g., Stewart, et al. (1984) Solid Phase Peptide Synthesis (2nd Ed.); and Chan (2000) "FMOC Solid Phase Peptide Synthesis, A Practical Approach," Oxford University Press. Peptides may be synthesized using an automated peptide synthesizer (e.g., a Pioneer™ Peptide Synthesizer, Applied Biosystems, Foster City, CA). For example, a peptide may be prepared on Rink amide resin using FMOC solid phase peptide synthesis followed by trifluoroacetic acid (95%) deprotection and cleavage from the resin.

It will be readily apparent that the first and second peptides have different primary amino acid sequences.

It will be further apparent that the first and second peptides can be synthesised from first and second amino acid sets and that each amino acid set may be the same or different.

Each peptide further comprises a reactive group. The reactive groups may be protected during peptide synthesis and deprotected prior to use in production of capture agents.

Such techniques are well known to those skilled in the art, for example, standard FMOC-based solid-phase peptide assembly can be used. In this technique, resin bound peptides with protected side chains and free amino termini are generated. The amino groups at the N-terminus may then be reacted with any compatible carboxylic acid/reactive group conjugate under standard peptide synthesis conditions. For example, cysteine with a trityl or methoxytrityl protected thiol group could be incorporated. Deprotection with trifluoroacetic acid would yield the unprotected peptide in solution.

The reactive groups are, thiol groups.

It will be understood that any suitable reaction may be used to form the peptide multimers, for example, Diels Alder reaction between e.g. cyclopentadienyl functionalised peptides and maleimide functionalised peptides, Michael reaction between a thiol functionalised peptide and a maleimide functionalised peptide, reaction between a thiol functionalised peptide and a peptide containing an activated thiol group (activated with, for example, a (nitro)thiopyridine moiety) to form a disulfide, Staudinger ligation between an azide functionalised peptide and a phosphinothioester functionalised peptide, and native chemical ligation between a thioester and a N-terminal cysteine.

Dimers may be constructed wherein the first and second peptides each have a thiol group (which may or may not be activated) located at a site in the peptide sequence that is on the N-terminal side of the ligand-binding site or on the C-terminal side of the ligand-binding site or located internally within a bipartite ligand-binding site. In any of these embodiments, it will be clear that the thiol group moieties may have the same or opposite orientation as the ligand binding residues and that the location of each thiol group in the first and second peptide is independent.

It will be apparent that, depending upon the amino acid residues present in the peptides, the capture agents will have different characteristics. For example the side chains may provide a positive charge for ligand binding. Preferably, the positive charge is provided by a lysyl residue (four CH² groups between the peptide chain and the positive charge), an ornithyl residue (three CH² groups between the peptide chain and the positive charge) or most preferably, a diaminobutyryl residue (with two CH² groups between the peptide chain and the positive charge).

The amino acid may alternatively provide a hydroxyl group capable of acting as a hydrogen bond donor and/or acceptor for ligand binding. Preferably, the hydroxyl group is provided by a seryl residue (one CH² group between the peptide chain and the OH group), or more preferably a homoseryl residue (with two CH² groups between the peptide chain and the OH group).

The amino acid may provide a hydrophobic moiety for ligand binding. Preferably an alanyl residue (no CH² group between the peptide chain and the methyl group) or more preferably, an aminobutyryl residue (with one CH² group between the peptide chain and the methyl group) provides the hydrophobic moiety.

Alternatively, the amino acid may provide a negative charge for ligand binding. Preferably, the negative charge is provided by a glutamyl residue (two CH² groups between the peptide chain and the carboxylate group), or more preferably, an aspartyl residue (one CH² group between the peptide chain and the carboxylate group).

It will be apparent to the skilled person that, depending upon the number and sequence of amino acids present in the peptide chain, each chain will have different characteristics. The peptides are produced from the set of amino acids in a combinatorial manner, as is well known in the art, such that all possible combinations of amino acids present in the set may be produced, for example, if there are N amino acids in the set and the peptide is of length L, the complete set will comprise N^{L} peptides.

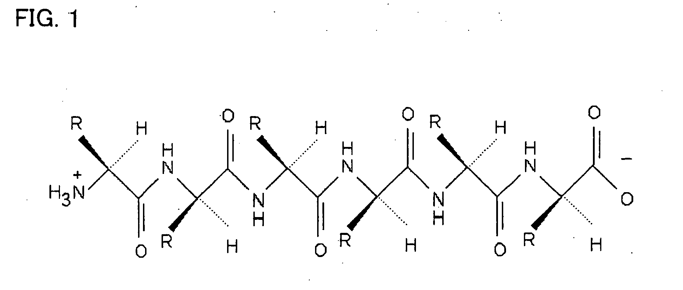

In a preferred embodiment, the peptides are produced such that the side chains are located in space in a manner which is favourable for ligand binding. This may be achieved by, for example, synthesising peptides having alternating L- and D-amino acids as shown in Figure 1.

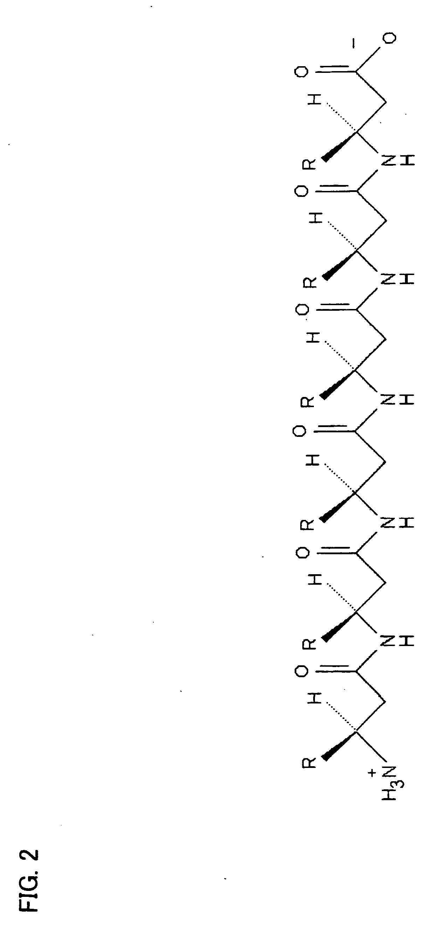

Alternatively, the peptides may be synthesised using a set comprising beta amino acids as shown in Figure 2, or any other chiral amino acid monomer with an even number of atoms per peptide repeat unit.

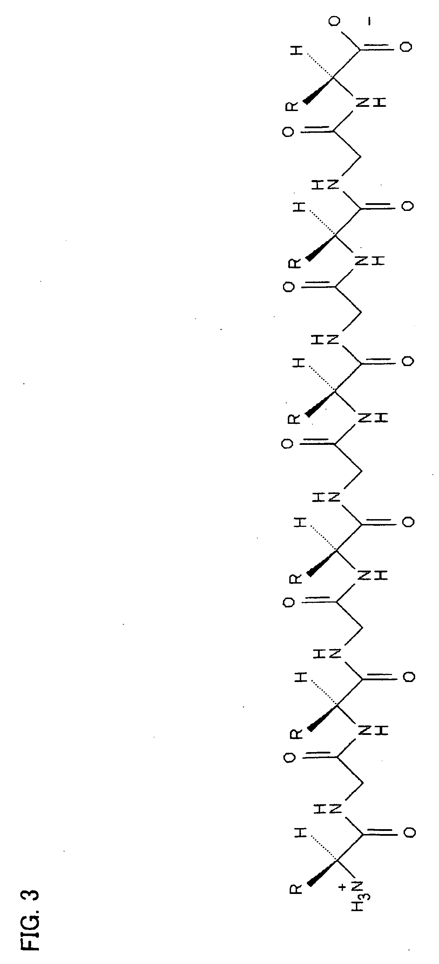

In a preferred embodiment, the peptides are synthesised such that only every second amino acid is varied, as shown in Figure 3.

This embodiment has the advantage that the side chains are spaced in the most natural and advantageous manner for ligand binding.

It will be obvious to the skilled person that each peptide can comprise one or more of the above types of amino acid and that the specific combinations employed will affect the characteristics of the capture agent containing the varying peptide chains.

It will be apparent that the increased diversity arises from the fact that the capture agents are dimmers. For example, because multimeric capture agent is a dimer, the possible diversity for any given length of peptide is squared due to the presence of two peptide chains.

In a preferred embodiment, a subset of the possible combinatorial peptides which can be produced from any given set of amino acids will be employed. The subset can be determined through the inclusion of specific rules in the synthesis of the peptide, for example, maximum and minimum levels of each amino acid in the set can be provided.

In a preferred embodiment, the combinatorially produced multimeric capture agents are bound to a substrate by an attachment moiety.

It will be understood that the attachment moiety may be any suitable moiety. It will be understood that attachment may be, for example, by covalent or hydrophobic interactions. In a preferred embodiment, the attachment moiety is a hydrophobic moiety. In another preferred embodiment, the attachemnt moiety is capable of forming a covalent bond, either directly or indirectly, where indirectly is understood to mean via an intermediate or following chemical modification of the attachment moiety.

Multiple capture agents are bound to the substrate so as to produce an array. It will be understood that the array may take any convenient form. Thus, the method described herein is applicable to all types of "high density" arrays, including single-molecule arrays.

In a particular preferred embodiment, the dimeric capture agents are assembled on the substrate.

The array comprises a number of discrete addressable spatially encoded locations. Each location on the array comprises a different capture agent, and more preferably, each location comprises multiple copies of the capture agent.

When referring to immobilisation of molecules (e.g. peptides) to a substrate, the terms "immobilised" and "attached" are used interchangeably herein and both terms are intended, to encompass direct or indirect, covalent or non-covalent attachment; unless indicated otherwise, either explicitly or by context. In certain embodiments of the invention, covalent attachment may be preferred but generally all that is required is that the molecules (e.g. peptides) remain immobilised or attached to the support under the conditions in which it is intended to use the support, for example in applications requiring ligand binding.

Certain embodiments of the invention may make use of solid supports comprised of an inert substrate or matrix (e.g. glass slides, polymer beads etc) which has been "functionalised", for example by application of a layer or coating of an intermediate material comprisng reactive groups which permit covalent attachment to biomolecules such as peptides. Examples of such supports include, but are not limited to, polyacrylamide hydrogels supported on an inert substrate such as glass. In such embodiments, the biomolecules. (e.g. peptides) may be directly covalently attached to the intermediate material (e.g. the hydrogel) but the intermediate material may itself be non-covalently attached to the substrate or matrix (e.g. the glass substrate). The term "covalent attachment to a substrate" is to be interpreted accordingly as encompassing this type of arrangement.

In multi-peptide arrays, distinct regions on the array comprise multiple peptide, molecules. Preferably, each site on the array comprises multiple copies of one individual peptide dimer.

Multi-peptide arrays of peptide molecules may be produced using techniques generally known in the art.

Also described herein is a method of producing a multimeric capture agent for binding a ligand, said capture agent comprising at least first and second peptides, said first peptide further comprising a first reactive group, said second peptide further comprising a second reactive group,
wherein the reactive groups may be the same or different for each peptide,
said method comprising the steps of;
reacting the first peptide with the second peptide such that the reactive groups present on the peptides react to form a covalently linked dimer.

It will be understood that further peptides having further reactive groups may be reacted with the first and / or second peptides to form higher order multimeric capture agents. It will also be understood that each peptide may include more than one reactive group such that it may react with a plurality of other peptides to form multimeric capture agents.

Preferably, the first and second peptides are synthesised on a solid phase, more preferably, the peptides are cleaved from the solid phase prior to use in the method of the second aspect.

Syntheses of peptides and their salts and derivatives, including both solid phase and solution phase peptide syntheses, are well established in the art. See, e.g., Stewart, et al. (1984) Solid Phase Peptide Synthesis (2nd Ed.); and Chan (2000) "FMOC Solid Phase Peptide Synthesis, A Practical Approach," Oxford University Press. Peptides may be synthesized using an automated peptide synthesizer (e.g., a Pioneer™ Peptide Synthesizer, Applied Biosystems, Foster City, CA): For example, a peptide may be prepared on Rink amide resin using FMOC solid phase peptide synthesis followed by trifluoroacetic acid (95%) deprotection and cleavage from the resin.

It will be readily apparent that the first and second peptides can have the same or different primary amino acid sequences.

It will be apparent that the first and second peptides can be synthesised from first and second amino acid sets and that each amino acid set may be the same or different.

Preferably, said peptides are between 2 and 100 amino - acids in length, more preferably, 2 to 50 amino acids in length, and most preferably, 5 to 25 amino acids in length.

Preferably, each amino acid is selected from a set consisting essentially of less than 20 amino acids, more preferably less than 12 amino acids, even more preferably less than 6 amino acids and most preferably 4 amino acids.

It will be understood that each amino acid in the set may comprise any of the following monomers, with the proviso that each monomer is substantially enantiomerically pure, an L-amino acid, a D-amino acid, an amino acid mimetic, a spacer amino acid, a beta amino acid, or any other chiral amino acid monomer. Preferably, the substantially enantiomerically pure amino acids are L-amino acids and/or D-amino acids.

In a preferred embodiment, said reactive groups may be protected during peptide synthesis and deprotected prior to use in the method of the second aspect. Such techniques are well known to those skilled in the art, for example, standard FMOC-based solid-phase peptide assembly. In this technique, resin bound peptides with protected side chains and free amino termini are generated. The amino groups at the N-terminus may then be reacted with any compatible carboxylic acid/ reactive group conjugate under standard peptide synthesis conditions. For example, cysteine with a trityl or methoxytrityl protected thiol group, could be incorporated. Deprotection with trifluoroacetic acid would yield the unprotected peptide in solution.

Preferably, said reactive groups are selected from, but not limited to thiol groups, maleimide, cyclopentadiene, azide, phosphinothioesters, thioesters, (nitro)thiopyridyl activated thiols and other such compounds known in the art. More preferably, the reactive groups are thiol groups.

It will be understood that any suitable reaction may be used to form the peptide multimers, for example, Diels Alder reaction between e.g. cyclopentadienyl functionalised peptides and maleimide functionalised peptides, Michael reaction between a thiol functionalised peptide and a maleimide functionalised peptide, reaction between a thiol functionalised peptide and a peptide containing an activated thiol group (activated with, for example, a (nitro)thiopyridine moiety) to form a disulfide, Staudinger ligation between an azide functionalised peptide and a phosphinothioester functionalised peptide, and native chemical ligation between a thioester and a N -terminal, cysteine.

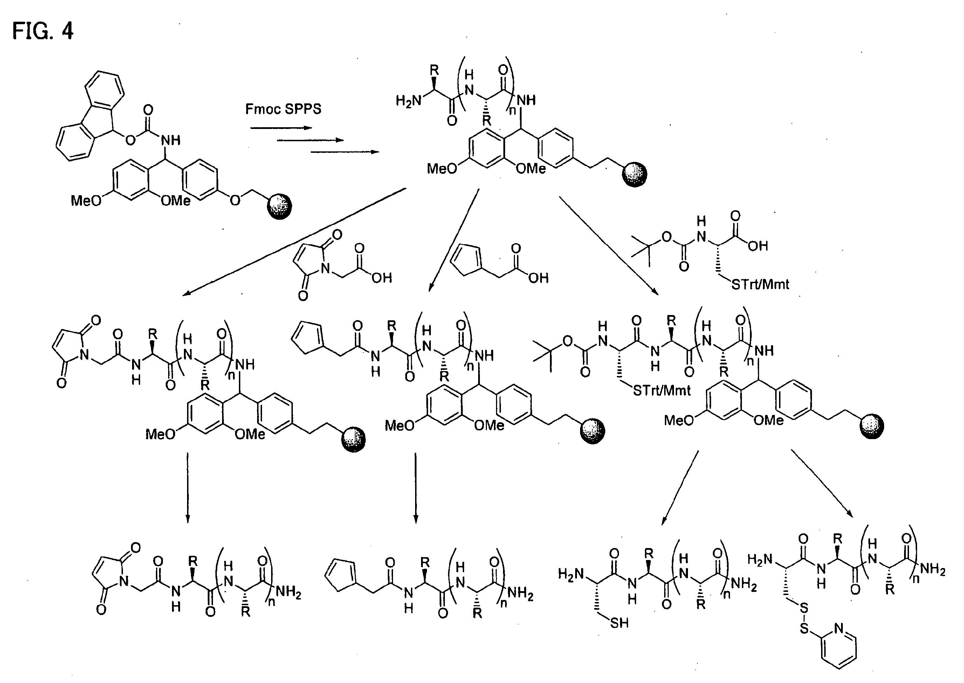

The reaction scheme outlined in Figure 4 shows a possible route for generating peptides comprising various reactive groups.

In preferred embodiments, the dimer may be constructed wherein the first and second peptides each have a thiol group (which may or may not be activated) located at a site in the peptide sequence that is on the N-terminal side of the ligand-binding site or on the C-terminal side of the ligand-binding site or located internally within a bipartite ligand-binding site. In any of these embodiments, it will be clear that the thiol group moieties may have the same or opposite orientation as the ligand binding residues and that the location of each thiol group in the first and second peptides is independent.

It will be apparent that, depending upon the amino acid residues present in the peptides, the capture agents will have different characteristics as discussed in relation to the first aspect.

Preferably, the capture agents produced by the method described herein are attached to a substrate. More preferably, the capture agents produced by the method described herein are attached to the substrate so as to form an array. It will be understood that the array may take any convenient form. Thus, the method is applicable to all types of "high density" arrays, including single-molecule arrays.

In a particularly preferred embodiment, the multimeric capture agents are assembled on the substrate.

Preferably, the array comprises a number of discrete addressable spatially encoded loci. Preferably, all of said capture agents at a given locus on the array are comprised of the same pairs of peptides. More preferably, each given locus on the array comprises a different capture agent.

When referring to immobilisation of molecules (e.g. peptides) to a substrate, the terms "immobilised" and "attached" are used interchangeably herein and both terms are intended to encompass direct or indirect, covalent or non-covalent attachment, unless indicated otherwise, either explicitly or by context. In certain embodiments of the invention, covalent attachment may be preferred, but generally all that is required is that the molecules (e.g. peptides) remain immobilised or attached to the support under the conditions in which it is intended to use the support, for example in applications requiring ligand binding.

Certain embodiments of the invention may make use of solid supports comprised of an inert substrate or matrix (e.g. glass slides, polymer beads etc) which has been "functionalised", for example by application of a layer or coating of an intermediate material comprising reactive groups which permit covalent attachment to biomolecules, such as peptides, Examples of such supports include, but are not limited to, polyacrylamide hydrogels supported on an inert substrate such as glass. In such embodiments, the biomolecules (e.g. peptides) may be directly covalently attached to the intermediate material (e.g. the hydrogel) but the intermediate material may itself be non-covalently attached to the substrate or matrix (e.g. the glass substrate). The term "covalent attachment to a substrate" is to be interpreted accordingly as encompassing this type of arrangement.

In multi-peptide arrays, distinct regions on the array comprise multiple peptide molecules. Preferably, each site on the array comprises multiple copies of one individual peptide dimer.

Multi-peptide arrays of peptide molecules may be produced using techniques generally known in the art.

Also described herein is a method of producing a multimeric capture agent for binding a ligand, said capture agent comprising at least first and second peptides,
said first peptide further comprising a first reactive group and an attachment moiety,
said second peptide further comprising a second reactive group,
wherein the reactive groups may be the same or different for each peptide,
said method comprising the steps of;
a) reacting the first peptide with the second peptide such that reactive groups present on the peptides react to form a multimeric structure; and
b) attaching the first peptide to a substrate via the attachment moiety,
wherein, step a) can be performed before, simultaneously with, or subsequently to step b).

Preferably, the first and second peptides are synthesised on a solid phase and can be the same or different, more preferably, the peptides are cleaved from the solid phase prior to use in the method.

Syntheses of peptides and their salts and derivatives, including both solid phase and solution phase peptide syntheses, are well established in the art. See, e.g., Stewart, et al. (1984) Solid Phase Peptide Synthesis (2nd Ed.); and Chan (2000) "FMOC Solid Phase Peptide Synthesis, A Practical Approach," Oxford University Press. Peptides may be synthesized using an automated peptide synthesizer (e.g., a Pioneer™ Peptide Synthesizer, Applied Biosystems, Foster City, CA). For example, a peptide may be prepared on Rink amide resin using FMOC solid phase peptide synthesis followed by trifluoroacetic acid (95%) deprotection and cleavage from the resin.

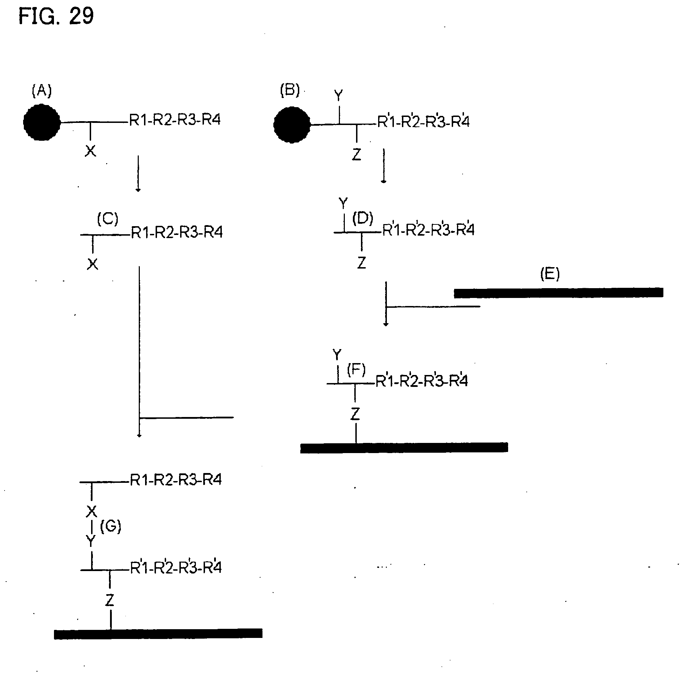

Figure 29 shows a schematic representation of a method of producing capture agents.

A first set of monomer units (A) is prepared on a solid phase. The monomer units comprise a ligand-binding moiety (R1 - R4) and a reactive group X. If wished X may be protected during synthesis and then deprotected before use.

A second set of monomer units (B) is prepared on a solid phase. These monomer units comprise a ligand-binding moiety (R'1 - = R'4), a reactive group Y, which may be protected during synthesis and then, deprotected before use, and an attachment moiety Z. If wished Z may also be protected during synthesis and then deprotected before use.

Each of the monomer units in set (A) is cleaved off the solid support to give monomer units (C) in solution.

Each of the monomer units in set (B) is cleaved off the solid support to give monomer unfits (D) in solution.

Each of the monomer units in set D is then contacted with the surface of a solid support (E) at a spatially encoded location in an array such that Z is used to bring about attachment to the said surface.

Reactions are then performed wherein surface-bond monomer units (F) from set D are reacted with an excess or equimolar amount of a given solution phase monomer unit (C) such that residue X reacts with residue Y to form a dimeric structure (G) bound to the solid phase.

The arrayed and spatially encoded dimeric structures (G) can then be used for binding to ligands of interest that will bind with suitable affinity, and selectivity.

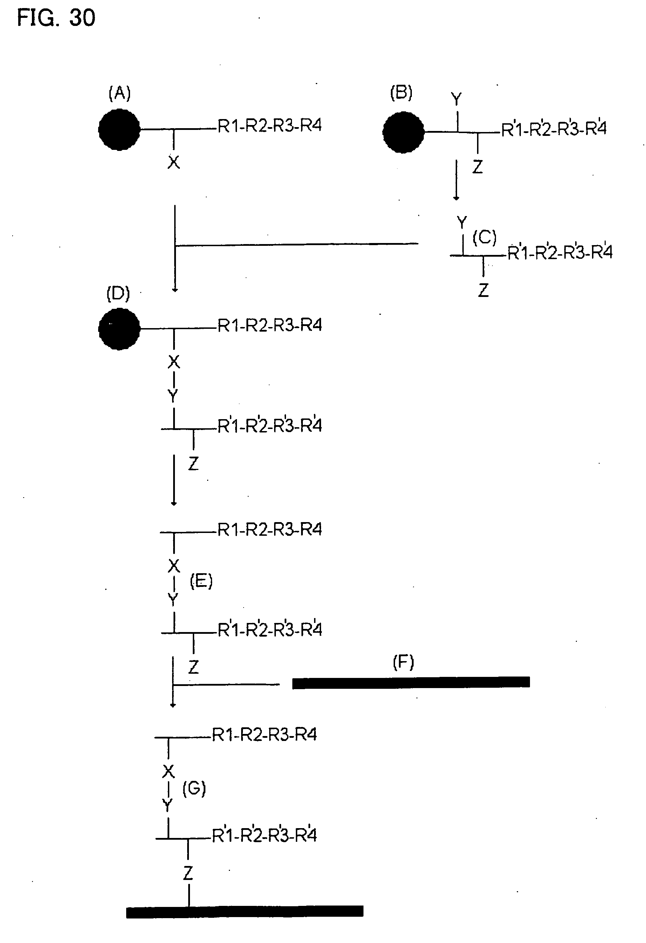

Figure 30 shows a schematic representation of a second method of producing capture agents.

In this embodiment, a first set of monomer units (A) is prepared on a solid phase. The monomer units comprise a ligand-binding moiety (Rl - R4) and a reactive group X, which may be protected during synthesis and then deprotected before use.

A second set of monomer units (B) is also prepared on a solid phase. These monomer units comprise a ligand-binding moiety (R'1 - R'4) and a reactive group Y, which may be protected during synthesis and then deprotected before use, and an attachment moiety Z. If wished Z may also be protected during synthesis and then deprotected before use.

Each of the monomer units (B) is cleaved off the solid support to give monomer units (C) in solution. Reactions are then performed wherein a given solid phase-bound monomer unit from set (A) is reacted with an excess or equimolar amount of a given solution phase monomer unit (C) such that residue X reacts with residue Y to form a dimeric structure (D) bound to the solid phase.

Each dimeric structure (D) bound to the solid phase is then cleaved off the solid support to give a solution phase dimeric structure (E).

Each solution phase dimeric structure (E) is finally contacted with a solid surface (F) at a spatially encoded location in an array such that group Z is used to attach the dimeric structure to the said surface.

The arrayed and spatially encoded dimeric structures (G) can then be used for binding to ligands of interest that will bind with suitable affinity, and selectivity.

The positions of X, Y, and Z in the figures are merely illustrative and should' not be seen as limiting to the invention.

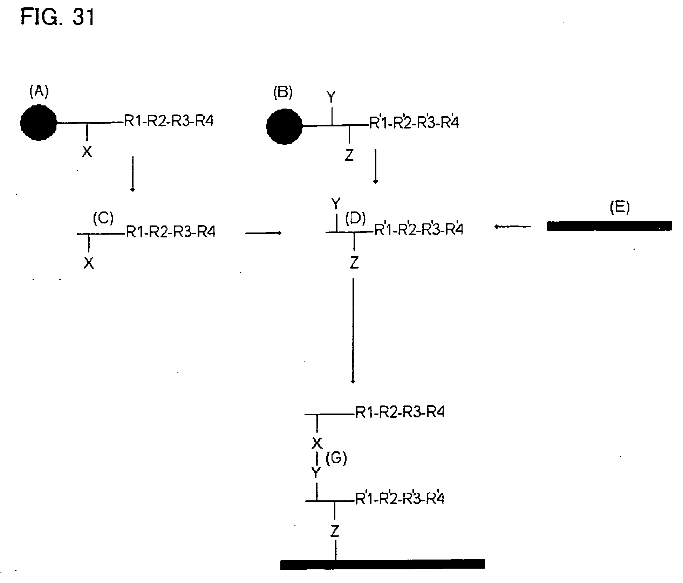

Figure 31 shows a schematic representation of a further method of producing capture agents.

A first set of monomer units (A) is prepared on a solid phase. The monomer units comprise a ligand-binding moiety (R1 R4) and a reactive group X. If wished X may be protected during synthesis and then deprotected before use.

A second set of monomer units (B) is prepared on a solid phase. These monomer units comprise a ligand-biding moiety (R'1 - R'4), a reactive group Y, which may be protected during synthesis and then deprotected before use, and an attachment moiety Z. If wished Z may also be protected during synthesis and then deprotected before use.

Each of the monomer units in set (A) is cleaved off the solid support to give monomer units (C) in solution.

Each of the monomer units in set (B) is cleaved off the solid support to give monomer units (D) in solution.

Each of the monomer units in set D is then contacted with an excess or equimolar amount of a given solution phase monomer unit (C) and the surface of a solid support (E) at a spatially encoded location in an array such that Z is used to bring about attachment to the said surface and such that residue X reacts with resid,ue Y to form a dimeric structure (G) bound to the solid phase.

The arrayed and spatially encoded dimeric structures (G) can then be used for binding to ligands of interest that will bind with suitable affinity, and selectivity.

The most significant advantage of the current invention is the 'squaring' (or raising to a higher power) of sequence diversity by the combinatorial joining of pairs (or greater numbers) of monomer units at the array surface.

The first and second peptides have different primary amino acid sequences.

It will be further apparent that the first and second peptides an be synthesised from first and second amino acid sets and that each amino acid set may be the same or different.

Each amino acid is selected from a set consisting essentially of less than 6 amino acids and most preferably 4 amino acids.

It will be understood that each amino acid in the set can be an L-amino acid or a D-amino acid. Preferably, the amino acids are L-amino acids and/or D-amino acids.

It will be understood that each amino acid in the set is substantially enantiomerically pure.

In a preferred embodiment, said reactive group may be protected during synthesis and deprotected as previously described prior to use in the method.

Preferably, said reactive groups are thiol groups.

It will be understood that any suitable reactive may be used to form the peptide dimers, for example, Diels Alder reaction between e.g. cyclopentadienyl functionalised peptides and maleimide functionalised peptides, Michael reaction between a thiol functionalised peptide and a maleimide functionalised peptide, reaction between a thiol functionalised peptide and a peptide containing an activated thiol group (activated with, for example, a (nitro)thiopyridine moiety) to form a disulfide, Staudinger ligation between an azide functionalised peptide, and native chemical ligation between a thioester and a N-terminal cysteine.

It will be apparent that, depending upon the amino acid residues present in the peptides, the capture agents will have different.

In preferred embodiments, the dimer may be constructed wherein the first and second peptides each have a thiol group (which may or may not be activated) located at a site in the peptide sequence that is on the N-terminal side of the ligand binding site or on the C-terminal side of the ligand-binding site. In any of these embodiments, it will be clear that the thiol group moieties have the same or opposite orientation as the ligand binding site and that the location of each thiol group in the first and second peptide is independent.

It will be understood that attachment may be, for example, by covalent or hydrophobic interactions. In a preferred embodiment, the attachment moiety is a hydrophobic moiety. In another preferred embodiment, the attachment moiety is capable of forming a covalent bond, either directly or indirectly, where indirectly is taken to mean via an intermediate or following chemical modification of the attachment moiety.

In a particularly preferred embodiment, the dimeric capture agents are assembled on the substrate.

If the capture agents are" arrayed" on a substrate, then the array may take any convenient form. Thus, the method is applicable to all types of "high density" arrays, including single-molecule arrays.

Preferably, the capture agents are located at discrete spatially encoded loci on an array. Preferably, all of said capture agents at a given locus on the array are comprised of the same pairs of peptides. Each locus on the array comprises a different capture agent.

When referring to immobilisation of molecules (e.g. peptides) to a substrate, the terms "immobilised" and "attached" are used interchangeably herein and both terms are intended to encompass direct or indirect, covalent or non-covalent attachment, unless indicated otherwise, either explicitly or by context. In certain embodiments of the invention covalent attachment may be preferred, but generally all that is required is that the molecules (e.g. peptides) remain immobilised or attached to the support under the conditions in which it is intended to use--the support, for example in applications requiring ligand binding.

Certain embodiments of the invention may make use of solid supports comprised of an inert substrate or matrix (e.g. glass slides, polymer beads etc) which has been "functionalised", for example by application of a layer or coating of an intermediate material comprising reactive groups which permit covalent attachment to biomolecules, such as peptides. Examples of such supports include, but are not limited to, polyacrylamide hydrogels supported on an inert substrate such as glass. In such embodiments, the biomolecules (e.g. peptides) may be directly covalently attached to the intermediate material (e.g. the hydrogel) but the intermediate material may itself be non-covalently attached to the substrate or matrix (e.g. the glass substrate). The term "covalent attachment to a substrate" is to be interpreted accordingly as encompassing this type of arrangement:

In multi-peptide arrays, distinct regions on the array comprise multiple peptide molecules. Preferably, each site on the array comprises multiple copies of one individual peptide dimer.

Multi-peptide arrays of peptide molecules may be produced using techniques generally known in the art.

The capture agents described herein can be attached to any suitable substrate by any suitable method. In a preferred embodiment, the capture agents are attached covalently to the substrate.

Preferred reaction schemes for the covalent, attachment of capture agents to substrates include, but are riot limited to; reaction between sulfhydryls and maleimide derivatised surfaces, Diels-Alder reaction between maleimide derivatised surfaces and diene functionalised capture agents., azide and acetylene 3+2 cycloaddition, thiazolidine ring formation, and the modified Staudinger ligation. Alternatively, covalent attachment of the capture agents to the substrates may be effected in the reverse fashion, for example by reaction between a thiol derivatised surface and maleimide substituted peptide.

In a particularly preferred embodiment, native chemical ligation between thioester-derivatised capture agents and cysteine-derivatised surfaces that present both the amino group and the sulfhydryl group of the cysteine is used to covalently link the capture agents to the substrate.

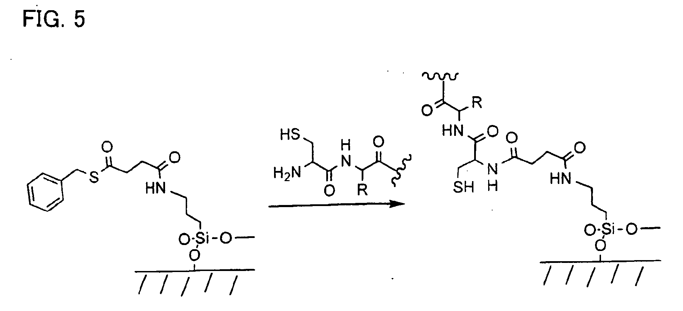

The most preferable reaction scheme uses native chemical ligation between capture agents with N-terminal cysteines and thioester-derivatised surfaces as shown in Figure 5.

Native chemical ligation generates a peptide bond between an N-terminal cysteine on a peptide and a surface-attached thioester: A particular advantage of this embodiment of the current invention is that protection of peptide side chains is not required. A further particular advantage of this embodiment of the current invention is that the resulting surface-attached peptides has an internal cysteine that may be exploited for the formation of dimeric preceptors by disulfide bond formation, or reaction between a thiol and a maleimide functionalised peptide.

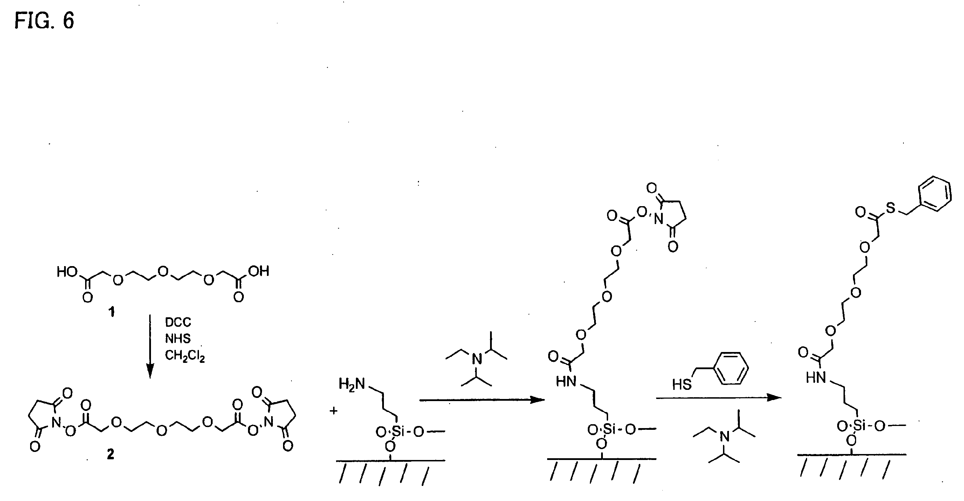

The preferred reaction scheme for the preparation of thioester functionalised glass surfaces is shown in Figure 6.

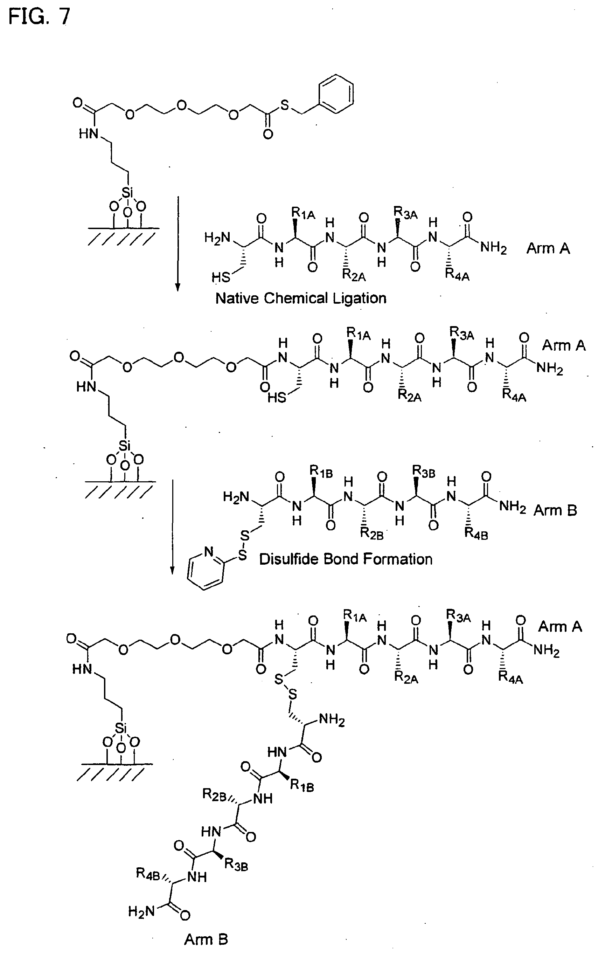

It will be apparent that the multimeric capture agent as described herein can be produced prior to covalent attachment to a substrate, or- may be assembled on the substrate itself. If the capture agent is assembled on the substrate surface, a preferred reaction scheme is shown in Figure 7. In this embodiment, the first peptide is covalently bound to the functionalised substrate via reaction between a thioester group and an N-terminal. cysteine residue via native chemical ligation. The dimene capture agent is produced by formation of disulfide bonds between the peptides.

A more preferable route for the preparation of thioester surfaces involves the reaction between an aminated surface and thiolane 2,4-diones of the type shown below:

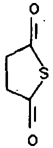

Thioester surfaces may also be made by derivatising hydroxylated surfaces with thioester silylchloride conjugates of the type shown below.

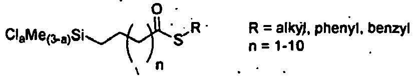

Also described herein, is a capture agent for binding a ligand, comprising at least first and second peptides, the first peptide comprising a plurality of hydrophobic amino acid residues and a plurality of non hydrophobic amino acid residues, wherein the amino acids are positioned in the peptide primary structure such that the peptide side chains are located to produce a hydrophobic face and a substantially non hydrophobic ligand-binding face and the second peptide comprising at least one hydrophobic amino acid residue and a plurality of non hydrophobic amino acid residues, wherein said amino acids are positioned in the peptide primary structure such that the amino acid side chains are located to produce a hydrophobic face and a substantially non hydrophobic ligand-binding face.

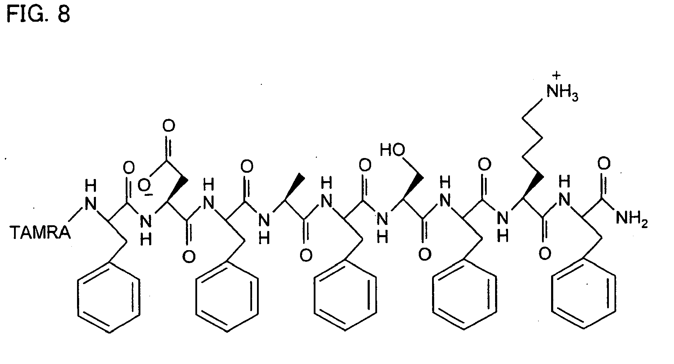

Preferably, the first peptide comprises a primary structure comprising alternating hydrophobic and non hydrophobic amino acid residues, as shown in Figure 8.

It will be understood by the skilled person that other peptide sequences which result in distribution of the side chains so as to result in a hydrophobic and substantially non hydrophobic face can be easily designed, for example, there may be three non hydrophobic amino acid residues between hydrophobic residues, or any combination of odd numbers of amino acid. Alternatively, the peptide may comprise a combination of, for example, L- D- and beta- amino acids so as to result in a hydrophobic and a substantially non hydrophobic face.

Preferably, the first peptide comprises 4 to 40 hydrophobic amino acid residues, more preferably 6 to 25 and most preferably 6 to 12.

Preferably, each amino acid positioned so as to be located on the ligand-binding face is selected from a set consisting essentially of less than 20 amino acids, more preferably, less than 12 amino acids, even more preferably less than 6 amino acids and most preferably 4 amino acids.

It will be understood that each amino acid monomer can be an L-amino acid, a D-amino acid, an amino acid mimetic, a spacer amino acid, a beta amino' acid, or any other chiral amino acid monomer. Preferably; amino acids' are L-amino acids and/or D-amino acids.

Preferably, each amino acid monomer is substantially enantiomerically pure.

It will be understood that amino acids positioned on the ligand-binding face may also include hydrophobic residues, for example, aminobutyrate residues.

Preferably, the first peptide comprises 10% to 90% hydrophobic amino acid residues, more preferably, 20% to 80%, even more preferably, 30% to 70%, and most preferably 40% to 60% hydrophobic amino acid residues.

In a particularly preferred embodiment, the first peptide comprises 50% hydrophobic amino acid residues.

Preferably, the hydrophobic amino acids which form the hydrophobic face are selected from the group consisting of leucine, isoleucine, norleucine, valine, norvaline, methionine, tyrosine, tryptophan and phenylalanine. More preferably, the hydrophobic amino acids are phenylalanine.

In a preferred embodiment, the capture agent is located on a hydrophobic substrate such that the substantially non hydrophobic ligand-binding face is accessible for ligand binding.

Preferably, the capture agent is bound to the hydrophobic substrate by a hydrophobic interaction between the substrate and the hydrophobic face of the peptide.

It will be understood that the substrate may be any suitable hydrophobic substrate, for example, gold modified by hydrophobic organic thiol treatment, glass modified by surface treatment, or plastic. Preferably, the substrate is plastic.

Alternatively, the substrate may be coated in a hydrophobic compound which allows the capture agents to be immobilised thereon in the presence of a substantially aqueous solvent.

It will be apparent that the peptide dimer can' be assembled from the first and second peptides before, simultaneously with or after the first peptide has been contacted with the hydrophobic substrate. In a particularly preferred embodiment, the peptide dimer is assembled on the hydrophobic substrate.

In a preferred embodiment, the second peptide comprises fewer amino acids than the first peptide, and contains fewer hydrophobic residues such that the interaction between the peptide and the hydrophobic surface is relatively weak. In this embodiment, the second peptide is only retained on the hydrophobic substrate when dimerised to the first peptide.

It will be apparent to the skilled person that the length of the first and second peptides and the numbers of hydrophobic amino acid residues required to retain them on the substrate will depend upon the hydrophobicity of the surface and on the hydrophobic amino acids present in the first and second peptides, and also on the nature of the ligand to be bound.

It will also be readily apparent to the skilled person that the amount of peptide retained at the substrate will depend upon the stringency of washing to which the substrate is subjected. Preferably, after immobilization of the peptides, the substrate is washed with, for example, 1.0 M NaCl in 10 mM tris-HCl (pH 8.0).

Preferably, the second peptide comprises 1-6 hydrophobic amino acid residues, more preferably, 2-5, and most preferably 2-4 hydrophobic amino acid residues one the hydrophobic face.

Preferably, the first and second peptides each contain 10 or fewer ligand-binding residues located on the substantially non hydrophobic ligand-binding face; more preferably, 8 or fewer; more preferably, 6 or fewer; even more preferably, 4 or fewer; and most preferably 3 or fewer.

Preferably, the capture agents described herein are produced from the sets of amino acids in a combinatorial manner as is well known in the art. Preferably, the peptides are produced to a set of rules, resulting in peptides having varied ligand-binding characteristics.

Preferably, the first and seconde peptides are synthesised on a solid phase, more preferably, the peptides are cleaved from the solid phase prior to use in the fourth aspect.

Synthesis of peptides and their salts and derivatives, including both solid phase and solution phase peptide syntheses, are well established in the art. See, e.g., Stewart, et al. (1984) Solid Phase Peptide Synthesis (2nd Ed.); and Chan (2000) "FMOC Solid Phase Peptide Synthesis, A Practical Approach," Oxford University Press. Peptides may 'be synthesized using an automated peptide synthesizer (e.g., a Pioneer™ Peptide Synthe,sizer, Applied Biosystems, Foster City, CA). For example, a peptide may be prepared on Rink amide resin using FMOC solid phase peptide synthesis followed by trifluoroacetic acid (95%) deprotection and cleavage from the resin.

It will be readily apparent that the at least first and second peptides can have the same or different primary amino acid sequences.

It will be further apparent that the first and second peptides can be synthesised from first and second amino acid sets and that each amino acid set may be the same or different.

Said first and second peptides each contain at least one reactive group. The reactive groups present on the peptides react so as to result in the formation of a multimeric capture agent.

In a preferred embodiment, said reactive groups may be protected during peptide synthesis and deprotected prior to use in production of capture agents according to the fourth aspects. Such techniques are well known to those skilled in the art, for example, standard FMOC-based solid-phase peptide assembly" In this technique, resin bound peptides with protected side chains and free amino, termini are generated. The amino groups at the N-terminus may then be reacted with any compatible carboxylic acid reactive group conjugate under standard peptide synthesis conditions. For example, cysteine with a trityl or methoxytrityl protected thiol group could be incorporated. Deprotection with trifluoroacetic acid would yield the unprotected peptide in solution.

It will be understood that any suitable reaction may be used to form the peptide multimers, for example, Diels Alder reaction between e.g. cyclopentadienyl functionalised peptides and maleimide functionalised peptides, Michael reaction between a thiol functionalised peptide and a maleimide functionalised peptide, reaction between a thiol functionalised peptide and a peptide containing an activated thiol group (activated with, for example, a (nitro) thiopyridine moiety) to form a disulfide, Staudinger ligation between an azide functionalised peptide and a phosphinothioester functionalised peptide, and native chemical ligation between a thioester and a N-terminal cysteine.

The peptide multimers are formed by disulphide bond formation.

It will be understood that the reactive groups may be located in the primary peptide structure of the first and second peptides at any suitable position, for' example, the reactive groups may be positioned in the primary peptide sequence such that they are positioned on the substantially non hydrophobic ligand-binding face of the peptides and located on the N-terminal side of the ligand-binding site.

Alternatively, the reactive groups may be located in the primary peptide structure of the first and second peptides such that they are positioned on the substantially non hydrophobic ligand-binding face of the peptides, and in the first peptide, on the N-terminal side of the ligand-binding site, and in the second peptide to the C-terminal side of the ligand-binding site.

In a further embodiment, the reactive group may be located in the primary peptide structure of the first and second peptides such that in the first peptide, it is positioned on the substantially non hydrophobic ligand-binding face of the peptides and to the N-terminal side of the ligand-binding site, and in the second peptide it is located on the opposite (hydrophobic) face to the ligand-binding site and to the C-terminal side of the ligand-binding site.

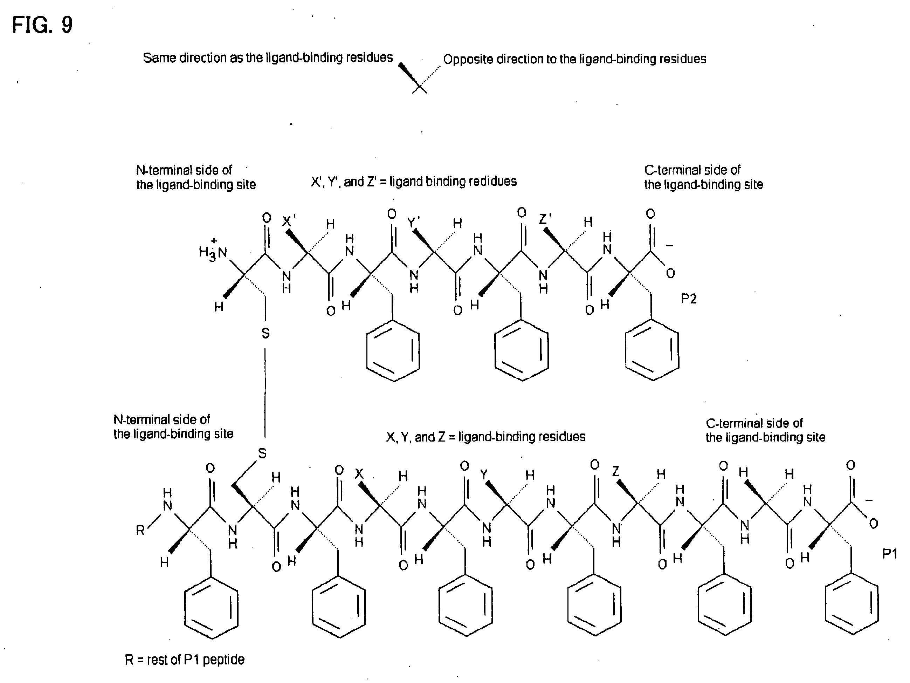

In a preferred embodiment, the reactive group on the first peptide is located in the primary amino acid structure on the substantially non hydrophobic ligand-binding face and to the N-terminal side of the ligand-binding site and in the second peptide, in the hydrophobic face and to the No-terminal side of the ligand-binding site as shown in Figure 9.

Preferably, said reactive groups are selected from, but not limited to, thiol groups, maleimide, cyclopentadiene, azide, phosphinothioesters, thioesters and (nitro)thiopyridyl activated thiols and other such compounds known in the 'art. More preferably, the reactive groups are thiol groups. Preferably, when the reactive groups are thiol groups, at least one thiol group is an activated thiol. Preferably, the thiol group is activated with either a nitrothiopyridyl or thiopyridyl group.

It will be apparent that, depending upon the amino acid residues present in the peptides, the capture agents will have different characteristics.

Preferably, the capture agents described herein are bound to the substrate so as to produce an array. It will be understood that the array may take any convenient form.

Thus, the method is applicable to all types of "high density" arrays, including single-molecule arrays.

Preferably, the array comprises a number of discrete addressable spatially' encoded loci. Preferably, each locus on the array comprises a different capture agent, and more preferably each locus comprises multiple copies of the capture agent.

In a particularly preferred embodiment, the first peptide has the structure set out in SEQ ID No 1;

(Phe-Gly)ₙ-Phe-Cys-Phe-X-Phe=Y-Phe-Z-Phe-Gly-Phe

where X, Y, and Z are the ligand-binding residues and Cys provides a nucleophilic thiol used for dimer formation.

The second peptide has the preferred structure set out in SEQ ID No 2;

CysS(N)P-X'-Phe-Y'-Phe-Z'-Phe

where X', Y', and Z' are the ligand-binding residues and CysS(N)P is an activated thiol used for dimer formation (most preferably activated with either a thionitropyridyl group or a thiopyridyl group).

It is to be understood that the preceding preferred embodiment is by way of example only and is not to be taken to be limiting. It will be apparent to the skilled person that many other reactive groups and activating groups can be employed in the current invention.

In the most preferred embodiment, the capture agents described herein are dispensed onto a suitable substrate to form an addressable spatially encoded array of combinatorially varying dimers. Preferably, the peptides are individually dispensed on to the substrate using a non-contact dispenser (eg. Piezorray System, Perkin Elmer LAS) and assembled in situ.

Also described herein is a substrate on which is immobilised at least one capture agent as described herein.

Also described herein is a substrate on which is immobilised at least one capture agent produced as described herein.

Also described herein is a method of identifying a multimeric capture agent which binds to a ligand of interest, said method comprising producing an array of combinatorial capture agents as described herein, contacting the ligand of interest with the array, and identifying to which capture agent(s) the ligand binds.

It will be apparent to the skilled person that the binding of the ligand to a capture agent can be identified in various ways known in the art, for example, the ligand or the capture agent may be labelled so that the location on the array to which the ligand binds can be identified. This label may be, for example, a radioactive or fluorescent label using, for example, fluorophores. Alternatively, binding of the ligand of interest to a capture agent may be detected by a variety of other techniques known in the art, for example, calorimetry, absorption spectroscopy, NMR methods, atomic force microscopy and scanning tunnelling microscopy, electrophoresis or chromatography, mass spectroscopy, capillary electrophoresis, surface plasmon resonance detection, surface acoustic wave sensing and numerous microcantilever-based approaches.

It will be understood that the arrays of dimeric capture agents of the current invention can be used to identify any analyte of choice, since the specific ligand which will be bound by the capture agent will be dependent upon the length and sequence of the peptides from which the capture agent is formed. In preferred embodiments the ligand comprises a eukaryotic cell, a prokaryotic cell, a virus, a bacteriophage, a prion, a spore, a pollen grain, an allergen, a nucleic acid, a protein, a peptide, a carbohydrate, a lipid, an organic compound, or an inorganic compound. The ligands are preferably physiological or pharmacological metabolites and most preferably, physiological or pharmacological metabolites in human or animal bodily fluids that may be used as diagnostic or prognostic healthcare markers.

Additional objects, features, and strengths of the present invention will be made clear by the d'escription below. Further, the advantages of the present invention will be evident from the following explanation in reference to the drawings.

### BRIEF DESCRIPTION OF DRAWINGS

The invention will be further understood with reference to the following experimental examples and accompanying figures in which:
Figure 1 shows a peptide comprising alternating L- and D- amino acids.
Figure 2 shows a peptide comprising beta amino acids.
Figure 3 shows a peptide wherein every second amino acid is varied.
Figure 4 shows a possible route for generating peptides comprising various reactive groups.
Figure 5 shows schematically the method of native chemical ligation between capture agents with N-terminal cysteines and thioester-derivatised surfaces.
Figure 6 shows the preferred reaction scheme for the preparation of thioester functionalised glass.
Figure 7 shows a preferred reaction scheme for the preparation of dimeric capture agents at a substrate surface.
Figure 8 shows a peptide comprising alternating hydrophobic and non hydrophobic amino acids.
Figure 9 shows an example of a dimeric capture agent having a hydrophobic face and a substantially non-hydrophobic ligand-binding face.
Figure 10 is a graphical representation showing the locations of various hydrophobic peptides in a 96 well plate.
Figure 11 shows fluorescence images of the 96 well plate of figure 10 indicating the presence of the various peptides in the wells.
Figure 12 shows a graphical representation of the quantified results of the 400V scan of Figure 11.
Figure 13A shows fluorescence images indicating the retention of polypeptides P1-1 to P1-5 and P2-1 to P2-2 on a polypropylene surface.
Figure 13B shows fluorescence images indicating the retention of polypeptides P1-1 to P1-5 and P2-1 to P2-2 on a polypropylene surface.
Figure 14 shows a graphical representation of the quantified results of Figure 13A,B.
Figure .15 shows fluorescence images indicating the pH resistance of the peptide 2DOS-2 deposited on to a polypropylene hydrophobic surface.
Figure 16 shows a graphical representation of the quantified results of the 300V scan of Figure 15.
Figure 17 shows fluorescence images indicating the time dependent persistence of the peptide 2DOS-2 deposited on to a polypropylene hydrophobic surface in the presence of an aqueous buffer.
Figure 18 shows a graphical representation of the results of the 300V scan of Figure 17.
Figure 19 is a graphical representation showing the location of various hydrophobic peptides added to flat bottomed and V-bottomed polypropylene 96 well plates.
Figure 20 shows fluorescence images of the plates of Figure 19 showing retention of the hydrophobic peptides with and without washing.
Figure 21 is a graphical representation of the results of the 500V scan of Figure 20 for the V-bottomed plates.
Figure 22 is a graphical representation of the results of the 500V scan of Figure 20 for the flat bottomed plates.
Figure 23 is a graphical representation showing the location of various hydrophobic peptides added to polypropylene and polystyrene V-bottomed 96 well plates.
Figure 24 shows fluorescence images of the plates of Figure 23 showing retention of the hydrophobic peptides with and without washing.
Figure 25 is a graphical representation showing the percentage retention of the various peptides in the polypropylene and polystyrene plates of Figure 23 after washing.
Figure 26A shows fluorescence images of the microtitre plate from the experiment using the liquid phase protocol.
Figure 26B is a graphical representation of the data from the fluorescence image shown in Table 21.
Figure 27A shows fluorescence images of the microtitre plate from the experiment using the `co-drying' protocol.
Figure 27B is a graphical representation of the data from the fluorescence image shown in Table 22.
Figure 28 shows fluorescence images indicating the yield of dimer formation on polypropylene sheets.
Figure 29 shows a schematic representation of a first method of fabricating a capture agent
Figure 30 shows a schematic representation of a second method of fabricating a capture agent.
Figure 31 shows a schematic representation of a third method of fabricating a capture agent.
Figure 32 shows a fluorescence images of a 256-element microarray of peptide dimers.

### BEST MODE FOR CARRYING OUT THE INVENTION

As used herein, the term spacer amino acid refers to an amino acid, a synthetics amino acid, an amino acid analogue or amino acid mimetic in which the side chains play no part in ligand binding.

As used herein, the term capture agent refers to a peptide molecule having a structure such that when a ligand is brought into contact with the capture agent it is bound thereto.

As used herein, the term multimeric capture agent refers to a capture agent comprising at least two linked subunits.

As used herein, the term peptide refers to a chain comprising 2 or more amino acid residues, Synthetic amino acids, amino acid analogues or amino acid mimetics, or any combination thereof. The term peptide and polypeptide are used interchangeably in this specification.

As used herein, the term substantially enantiomerically pure indicates that the residue comprises substantially one type of isomer, with any other isomeric forms being there only as an impurity.

As used herein, the term located in space in a manner favourable to ligand binding indicates that the side chains of the peptides which make up the multimeric capture agent are positioned such that they are able to contact and interact with a ligand.

As used herein, the term substantially non hydrophobic means comprising substantially more hydrophilic residues than hydrophobic residues.

### Example 1

The following series of peptides were synthesised in order to demonstrate peptide self-assembly into an organic solvent layer or onto a hydrophobic surface driven by entropic effects in an aqueous solvent in contact with the said organic solvent layer or hydrophobic surface.

All peptides are labelled with the rhodamine dye TAMRA at the N-terminus. A mixture of the 5-TAMRA and 6-TAMRA isomers was used for the labelling.

| 5-carboxytetramethylrhodamine (5-TAMRA) | 6-carboxytetramethylrhodamine (6-TAMRA) |
|---|---|
| 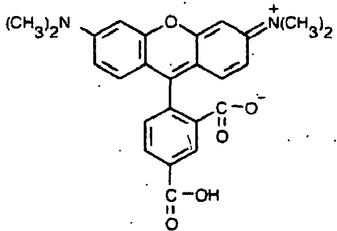 | 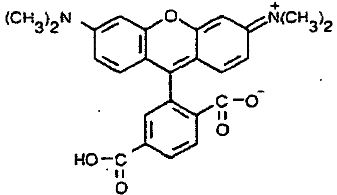 |

| Spectrum | Spectrum |
|---|---|
| 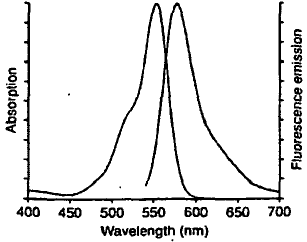 | 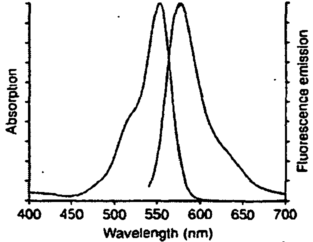 |

In the following, the residue side chains projecting in front of the plane of the paper represent the combinatorially varied 'ligand-binding face'. The residue side chains projecting behind the plane of the paper represent the 'hydrophobic face' (or negative control residues).

In the set of peptides 2DOS-1 to 2DOS-8, a mixture of four side chains (aspartyl, alanyl,' seryl, and lysyl) has been used. In the set of peptides 2DOS-9 to 2DOS-16, four hydrophilic (aspartyl) chains have been used.

In the set of peptides 2DOS-1 to 2DOS-4 and the set of peptides 2DOS-9 to 2DOS-12, five residue side chains have been used for the 'hydrophobic face' (or negative control residues). In the set of peptides 2DOS-5 to 2DOS-8 and the set of peptides 2DOS-13 to 2DOS-16, three residue side chains have been used for the 'hydrophobic face' (or negative control residues).

For peptides 2DOS-1, 2DOS-5, 2DOS-9, and 2DOS-13, norleucyl residues have been used for the 'hydrophobic face'. For peptides 2DOS-2, 2DOS-6, 2DOS-10, and 2DOS-14, phenylalanyl residues have been used for the 'hydrophobic face'. For peptides 2DOS-3, 2DOS-7, 2DOS-11, and 2DOS-15, seryl residues have been used as a weak negative control for the 'hydrophobic face'. For peptides 2DOS-4, 2DOS-8, 2DOS-12, and 2DOS-16, aspartyl residues have been used as a strong negative control for the 'hydrophobic face'.

**Table 1**

| Peptide name | Peptide sequence | Peptide structure |
|---|---|---|
| 2DOS-1 | N-TAMRA- | 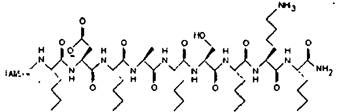 |
| | Norleu-Asp- | |
| | Norleu-Ala- | |
| | Norleu-Ser- | |
| | Norleu-Lys- | |
| | Norleu-C | |
| 2DOS-2 | N-TAMRA- | 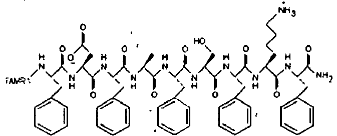 |
| | Phe-Asp- | |
| | Phe-Ala- | |
| | Phe-Ser- | |
| | Phe-Lys- | |
| | Phe-C | |
| 2DOS-3 | N-TAMRA- | 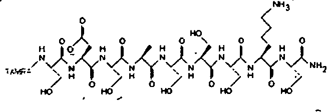 |
| | Ser-Asp- | |
| | Ser-Ala- | |
| | Ser-Ser- | |
| | Ser-Lys- | |
| | Ser-C | |
| 2DOS-4 | N-TAMRA-Asp-Asp-Asp-Ala -Asp-Ser-Asp-Lys-Asp-C | 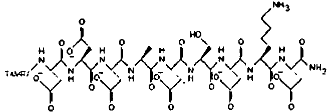 |
| 2DOS-5 | N-TAMRA- | 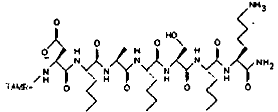 |
| | Asp-Norleu- | |
| | Ala-Norleu- | |
| | Ser-Norleu- | |
| | Lys-C | |
| 2DOS-6 | N-TAMRA- | 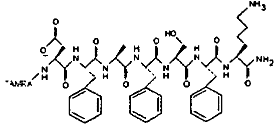 |
| | Asp-Phe- | |
| | Ala-Phe- | |
| | Ser-Phe- | |
| | Lys-C | |
| 2DOS-7 | N-TAMRA- | 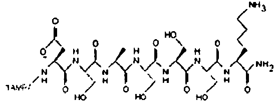 |
| | Asp-Ser- | |
| | Ala-Ser- | |
| | Ser-Ser- | |
| | Lys-C | |
| 2DOS-8 | N-TAMRA- | 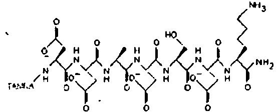 |
| | Asp-Asp- | |
| | Ala-Asp- | |
| | Ser-Asp- | |
| | Lys-C | |
| 2DOS-9 | N-TAMRA- | 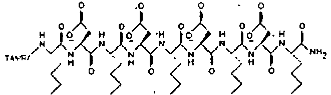 |
| | Norleu-Asp- | |
| | Norleu-Asp- | |
| | Norleu-Asp- | |
| | Norleu-Asp- | |
| | Norleu-C | |
| 2DOS-10 | N-TAMRA- | 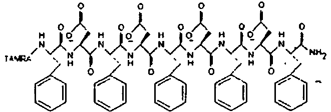 |
| | Phe-Asp- | |
| | Phe-Asp- | |
| | Phe-Asp- | |
| | Phe-Asp- | |
| | Phe-C | |
| 2DOS-11 | N-TAMRA- | 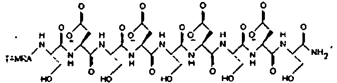 |
| | Ser-Asp- | |
| | Ser-Asp- | |
| | Ser-Asp- | |
| | Ser-Asp- | |
| | Ser-C | |
| 2DOS-12 | N-TAMRA- | 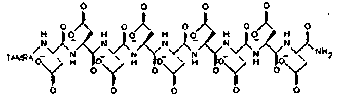 |
| | Asp-Asp- | |
| | Asp-Asp- | |
| | Asp-Asp- | |
| | Asp-Asp- | |
| | Asp-C | |
| 2DOS-13 | N-TAMRA- | 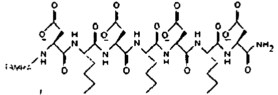 |
| | Asp-Norleu- | |
| | Asp-Norleu- | |
| | Asp-Norleu- | |
| | Asp-C | |
| 2DOS-14 | N-TAMRA- | 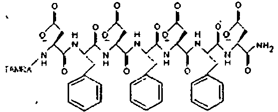 |
| | Asp-Phe- | |
| | Asp-Phe- | |
| | Asp-Phe- | |
| | Asp-C | |
| 2DOS-15 | N-TAMRA- | 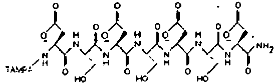 |
| | Asp-Ser- | |
| | Asp-Ser- | |
| | Asp-Ser- | |
| | Asp-C | |
| 2DOS-16 | N-TAMRA- | 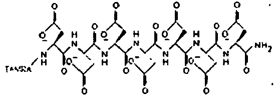 |
| | Asp-Asp- | |
| | Asp-Asp- | |
| | Asp-Asp- | |
| | Asp-C | |

Peptides were synthesised on a 2 µmol scale using standard FMOC chemistry (Alta Bioscience) and' were dissolved to 10 µM in 50% (v/v) aqueous acetonitrile.

The retention of peptides 2DOS-1 to 2DOS-16 on a hydrophobic surface (the wells of a polypropylene microtitre plate) was then investigated.

10 mM tris-HCl (pH 8.0) in 50% (v/v) aqueous acetonitrile was used as the solvent for the peptides and for TAMRA.

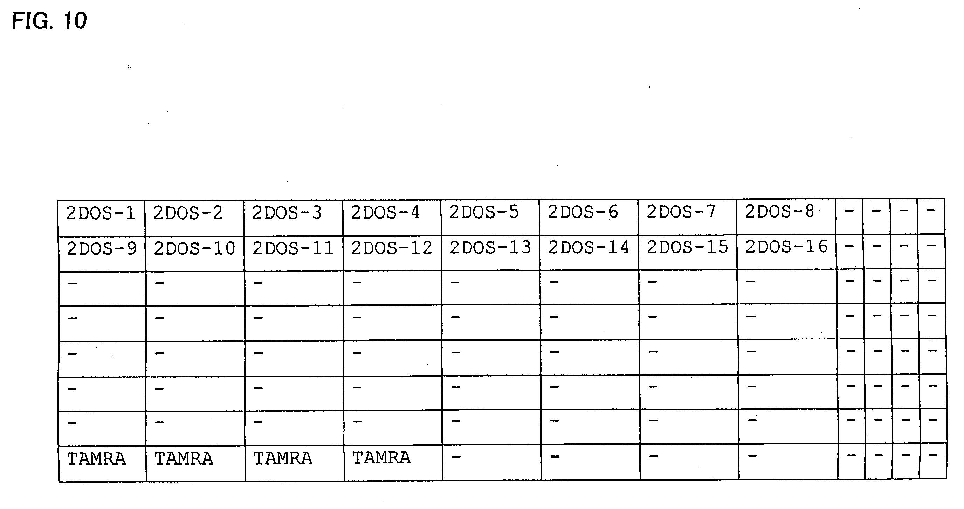

100 µl aliquots of 10 µM peptides 2DOS-1 to 2DOS-16 and 10 µM TAMRA were placed in the wells of a Costar microtitre plate as shown in Figure 10:

The microtitre plate was imaged at 200 µm resolution on a Typhoon Trio Plus variable mode imager (Amersham Biosciences) with the green (532 nm) laser and the 580 BP 30 filter at the PMT voltages indicated below and at normal sensitivity. The scan height was set at +3 mm and the sample was pressed during scanning.

The peptides were allowed to evaporate to dryness overnight in the dark and the microtitre plate was again scanned as described above.

The wells were then washed ten times with 250 µl of water.

The residual surface-bound peptides were finally resuspended in 100 µl of 10 mM tris-HCl (pH 8.0) in 50% (v/v) aqueous acetonitrile and the microtitre plate was again scanned as described above.

The fluorescence images were analysed using ImageQuant TL v2003.03 (Amersham Biosciences).

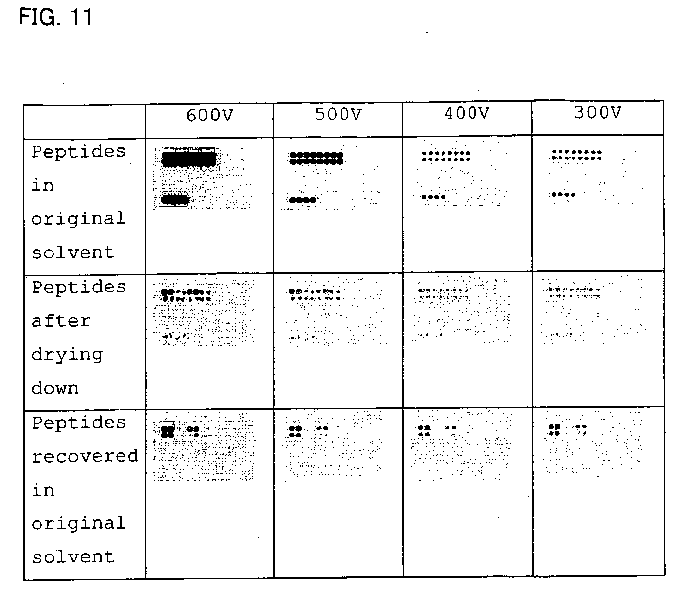

The fluorescence images of the microtitre plate scanned at PMT voltages of 600V, 500V, 400V, and 300V at the three stages of the experiment are shown in Figure 11:

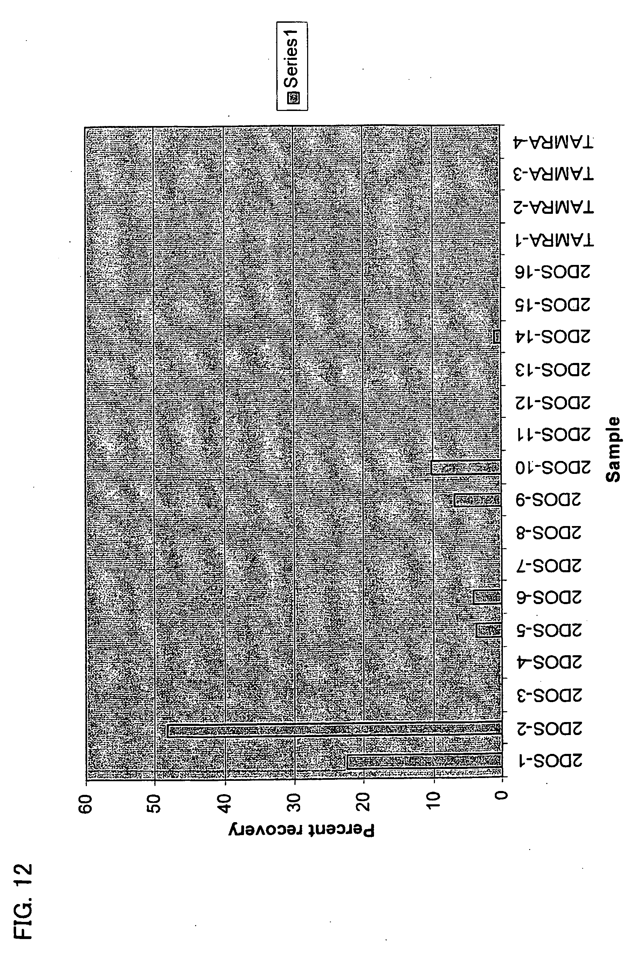

Quantification data (using the data from the 400V scan) is given in Table 2 and shown graphically in Figure 12.

**Table 2**

| Peptide | Initial fluorescence (x10³) | Recovered fluorescence (x10³) | Percent recovery |
|---|---|---|---|
| 2DOS-1 | 33,015 | 7,459 | 23 |
| 2DOS-2 | 32,492 | 15,704 | 48 |
| 2DOS-3 | 32,913 | 8 | 0 |
| 2DOS-4 | 32,313 | 0 | 0 |
| 2DOS-5 | 32,473 | 1,270 | 4 |
| 2DOS-6 | 33,853 | 1,455 | 4 |
| 2DOS-7 | 29,134 | 0 | 0 |
| 2DOS-8 | 33,615 | 3 | 0 |
| 2DOS-9 | 34,587 | 2,382 | 7 |
| 2DOS-10 | 28,479 | 2,884 | 10 |
| 2DOS-11 | 26,860 | 0 | 0 |
| 2DOS-12 | 26,433 | 1 | 0 |
| 2DOS-13 | 26,181 | 25 | 0 |
| 2DOS-14 | 28,071 | 283 | 1 |
| 2DOS-15 | 30,845 | 2 | -0 |
| 2DOS-16 | 30,335 | 3 | 0 |

The results show that phenylalanyl residues lead to greater retention than norleucyl residues. They also show that peptides with five hydrophobic `anchor residues' are retained better than equivalent peptides with three hydrophobic 'anchor residues'. Changing the `ligand-binding' residues from aspartyl, alanyl, seryl, and lysyl to a run of four aspartyl residues leads to a drop in retention on the polypropylene surface.

Further experiments were undertaken to investigate the retention of peptides P1-1 to P1-5 and P2-1 to P2-2, shown in Table 3, on a polypropylene surface.

**Table 3**

| Peptide | Sequence |
|---|---|
| P1-1 | TAMRA-F-G-F-S-F-A-F-D-F-G-F |
| P1-2 | TAMRA-F-G-F-G-F-S-F-A-F-D-F-G-F |
| P1-3 | TAMRA-F-G-F-G-F-G-F-S-F-A-F-D-F-G-F |
| P1-4 | TAMRA-F-G-F-G-F-G-F-G-F-S-F-A-F-D-F-G-F |
| P1-5 | TAMRA-F-G-F-G-F-G-F-G-F-G-F-S-F-A-F-D-F-G-F |
| P2-1 | TAMRA-G-S-F-A-F-D-F |
| P2-2 | TAMRA-G-S-G-A-F-D-F |

The polypropylene sheet was wiped with 50% (v/v) aqueous acetonitrile prior to use.

8x replicate 20 n1 volumes of 1 µM peptides P1-1 to P1-5 and, P2 to P2-2 and TAMRA in dimethyl sulphoxide (DMSO) were dispensed at 1 mm spacing to a 3"x1"x1 mm polypropylene sheet using the Piezorray system (PerkinElmer LAS). 500 drops were pre-dispensed using the 'side shoot' option and the tuning was set to 72V for 30µs.

The slide was imaged at 10 µm resolution on a Typhoon Trio Plus variable mode imager (Amersham Biosciences) with the green (532 nm) laser and the 580 BP 30 filter at a PMT voltage of 600 V and at normal sensitivity. The scan height was set at the platen and the samples were pressed during scanning. The fluorescence image was analysed using ImageQuant TL v2003.03 (Amersham Biosciences).

The lower half of the slide (containing the test array) was then washed in 100 ml of 1 M NaCl containing 10 mM tris-HCl (pH 8.0) for one minute and were re-scanned as described above.

The same half of the slide was then washed for a second time in 100 ml of 1 M NaCl containing 10 mM tris-HCl (pH 8.0) for 30 minutes and re-scanned as described above.

The same half of the slide was then washed for a third time in 100 ml of water for 30 minutes and re-scanned as described above.

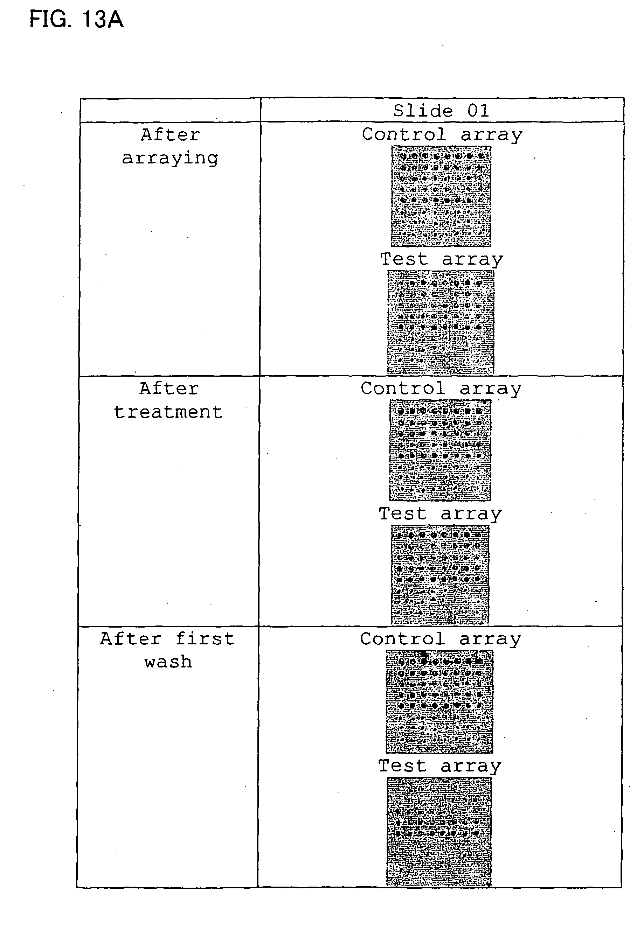

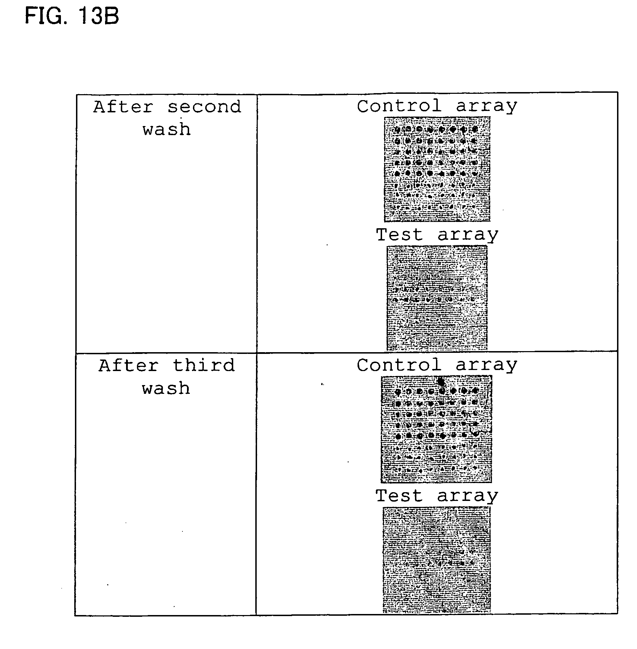

The fluorescence images for the various arrays are shown in Figure 13A,B.

The fluorescence values for the various arrayed peptides shown in Figure 13A,B are shown in Tables 4 - 6 below:

After first wash:

**Table 4a**

| Control array | | |
|---|---|---|
| Peptide | Average fluorescence | Corrected signal |
| P1-1 | 244,263,013 | 97,571,341 |
| P1-2 | 200,280,129 | 53,588,457 |
| P1-3 | 192,743,469 | 46,051,796 |
| P1-4 | 187,287,630 | 40,595,958 |
| P1-5 | 199,347,483 | 52,655,810 |
| | | |
| Average slide background = | 146,691,673 | |

**Table 4b**

| | | | |
|---|---|---|---|
| Test array | | | |

| Peptide | Average fluorescence | Corrected signal | Percentage recovery |
|---|---|---|---|
| P1-1 | 157,624,537 | 3,126,578 | 3 |
| P1-2 | 170,078,218 | 15,580,260 | 29 |
| P1-3 | 171,197,973 | 16,700,014 | 36 |
| P1-4 | 189,823,310 | 35,325,352 | 87 |
| P1-5 | 201,991,732 | 47,493,774 | 90 |
| | | | |
| Average slide background = | 154,497,959 | | |

After second wash:

**Table 5a**

| Control array | | |
|---|---|---|
| Peptide | Average fluorescence | Corrected signal |
| P1-1 | 225,863,933 | 88,584,083 |
| P1-2 | 196,080,891 | 58,801,042 |
| P1-3 | 187,310,655 | 50,030,806 |
| P1-4 | 179,942,418 | 42,662,569 |
| P1-5 | 190,847,825 | 53,567,975 |
| | | |
| Average slide background = | 137,279,849 | |

**Table 5b**

| Test array | | | |
|---|---|---|---|
| Peptide | Average fluorescence | Corrected signal | Percentage recovery |
| P1-1 | 127,216,792 | -5,230,512 | -6 |
| P1-2 | 135,695,639 | 3,248,336 | 6 |
| P1-3 | 148,725,456 | 16,278,152 | 33 |
| P1-4 | 159,100,527 | 26,653,223 | 62 |
| P1-5 | 167,865,473 | 35,418,169 | 66 |
| | | | |
| Average slide background = | 132,447,303 | | |

After third wash:

**Table 6a**

| Control array | | |
|---|---|---|
| Peptide | Average fluorescence | Corrected signal |
| P1-1 | 218,342,010 | 84,434,769 |
| P1-2 | 185,727,868 | 51,820,628 |
| P1-3 | 184,219,147 | 50,311,907 |
| P1-4 | 176,102,487 | 42,195,247 |
| P1-5 | 183,492,293 | 49,585,053 |
| | | |
| Average slide background = | 133,907,240 | |

**Table 6b**

| Test array | | | |
|---|---|---|---|
| Peptide | Average fluorescence | Corrected signal | Percentage recovery |
| P1-1 | 124,941,941 | 644,716 | 1 |
| P1-2 | 126,193,156, | 1,895,931 | 4 |
| P1-3 | 125,717,642 | 1,420,417 | 3 |
| P1-4 | 135,681,173 | 11,383,947 | 27 |
| P1-5 | 147,812,043 | 23,514,817 | 47 |
| | | | |
| Average slide background = | 124,297,225 | | |

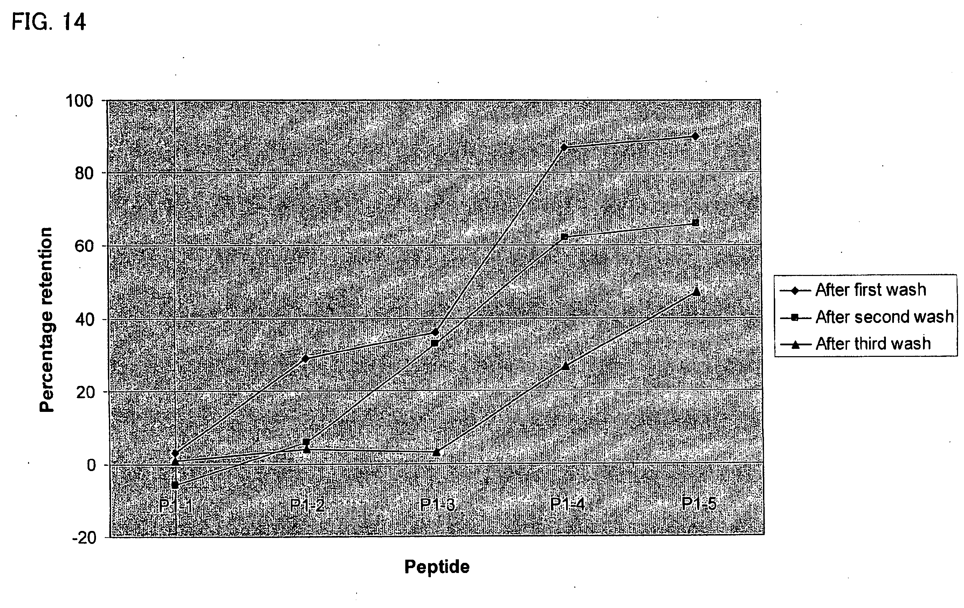

These results are shown graphically in Figure 14.

The figures clearly show that there is a gradient of increasing retention for the peptides correlated to increasing peptide chain length. As can be clearly seen, Peptide P1-5 which has a chain length of 19 amino acids has the highest retention.

### Example 2

The pH resistance of peptide 2DOS-2 (see above) deposited onto a polypropylene hydrophobic surface was investigated:

Twelve 50 µl aliquots of peptide 2DOS-2 in 10 mM tris-HCl (pH 8.0) in 50% (v/v) aqueous acetonitrile were dried down in the wells of a Costar microtitre plate.

The peptide samples were allowed to evaporate to dryness in the dark.

The dried peptide samples in the first eleven wells were incubated with 200 µl of 100 mM phosphate buffer for 30 minutes at room temperature according to the scheme shown in Table 7:

**Table 7**

| Well | µl of 100 mM NaH₂PO₄ | µl of 100 mM Na₂HPO₄ | Observed pH |
|---|---|---|---|
| 1 | 1,000 | 0 | 4.51 |
| 2 | 900 | 100 | 5.65 |
| 3 | 800 | 200 | 6.02 |
| 4 | 700 | 300 | 6.25 |
| 5 | 600 | 400 | 6.47 |
| 6 | 500 | 500 | 6.62 |
| 7 | 400 | 600 | 6.79 |
| 8 | 300 | 700 | 6.98 |
| 9 | 200 | 800 | 7.18 |
| 10 | 100 | 900 | 7.47 |
| 11 | 0 | 1,000 | 8.52 |

All supernatants were pipetted off and the residual surface-bound peptides in all twelve wells were finally resuspended in 50 µl of 10 mM tris-HCl (pH 8.0) in 50% (v/v) aqueous acetonitrile and the microtitre plate was imaged at 200 µm resolution on a Typhoon Trio Plus variable mode imager (Amersham Biosciences) with the green (532 nm) laser and the 580 BP 30 filter at the PMT voltages indicated below and at normal sensitivity. The scan height was set at +3 mm and the sample was pressed during scanning.

The fluorescence images were analysed using ImageQuant TL v2003.03 (Amersham Biosciences).

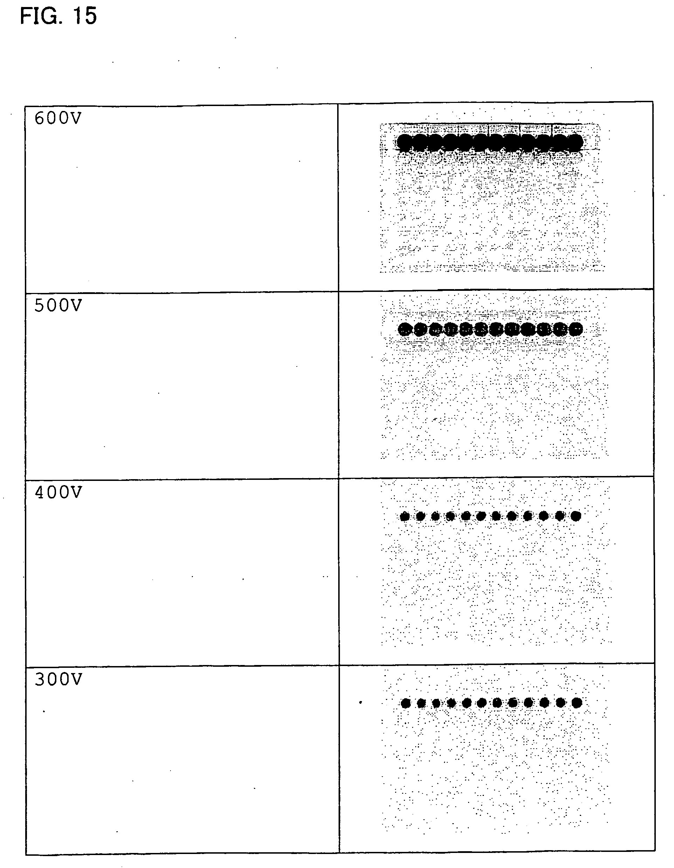

Fluorescence images showing the pH resistance of 2DOS-2 deposited on polypropylene are shown in Figure 15:

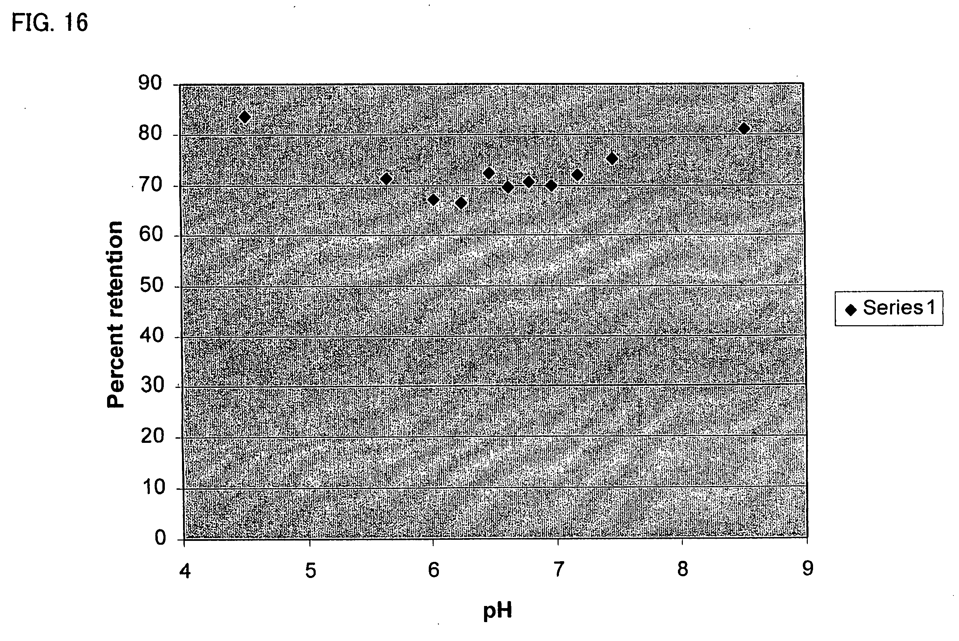

Quantification data for peptide 2DOS-2 retention '(using data from the 300V scan) are given in Table 8 and graphically in Figure 16:

**Table 8**

| Well | Wash pH | Untreated fluorescence | Retained fluorescence | Percent retention |
|---|---|---|---|---|
| 1 | 4.51 | 815,706 | 681,015 | 83 |
| 2 | 5.65 | 815,706 | 582,482 | 71 |
| 3 | 6.02 | 815,706 | 548,329 | 67 |
| 4 | 6.25 | 815,706 | 542,595 | 67 |
| 5 | 6.47 | 815,706 | 589,528 | 72 |
| 6 | 6.62 | 815,706 | 566,496 | 69 |
| 7 | 6.79 | 815,706 | 576,655 | 71 |
| 8 | 6.98 | 815,706 | 570,653 | 70 |
| 9 | 7.18 | 815,706 | 586,083 | 72 |
| 10 | 7.47 | 815,706 | 614,028 | 75 |
| 11 | 8.52 | 815,706 | 661,781 | 81 |

The results show that, the retention of peptide 2DOS-2 on a polypropylene surface is therefore stable over a broad range of pH values, with maximal retention at low and high pH and minimal retention around pH 6.5.

### Example 3

The time-dependent persistence of peptide 2DOS-2 (see above) deposited onto a polypropylene hydrophobic surface in the presence of aqueous buffer was investigated as shown below:

Twelve 100 µl aliquots of 5 µM peptide 2DOS-2 in 5 mM tris-HCl (pH 8.0) in 75% (v/v) aqueous acetonitrile where dispensed to the wells of the top row of a Costar microtitre plate.

The peptide samples were allowed to evaporate to dryness in the dark.

The dried peptide samples in wells 1-10 were incubated with 250 µl of 1 M NaCl in 10 mM tris-HCl (pH 8.0) for the time indicated below at room temperature. All-supernatants were pipetted up and down 8 times after incubation and the supernatants were then removed and placed in the wells of the bottom row of the microtitre plate.

The residual surface-bound peptides in all twelve wells were finally resuspended in 50 µl of 10 mM tris-HCl (pH 8.0) in 50% (v/v) aqueous acetonitrile and the microtitre plates were imaged at 200 µm resolution on a Typhoon Trio Plus variable mode imager (Amersham Biosciences) with the green (532 nm) laser and the 580 BP 30 filter at the PMT voltages indicated below and at normal sensitivity. The scan height was set at +3 mm and the sample was pressed during scanning.

The fluorescence images were analysed using ImageQuant TL v2003.03 (Amersham Biosciences).

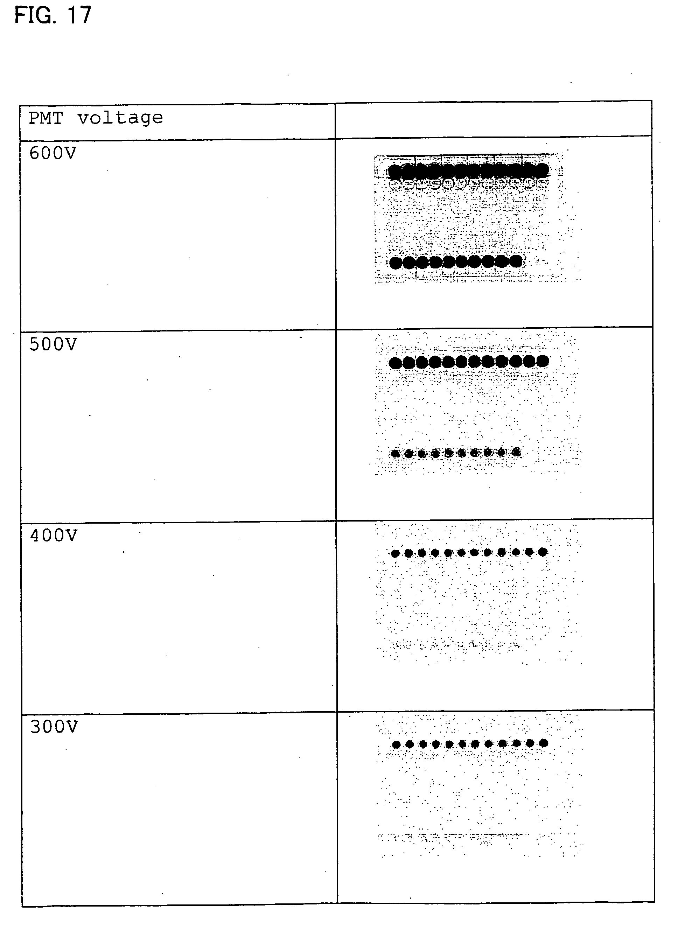

Fluorescence data for the time-dependent persistence of peptide 2DOS-2 deposited onto a polypropylene hydrophobic surface in the presence of aqueous buffer are shown in Figure 17:

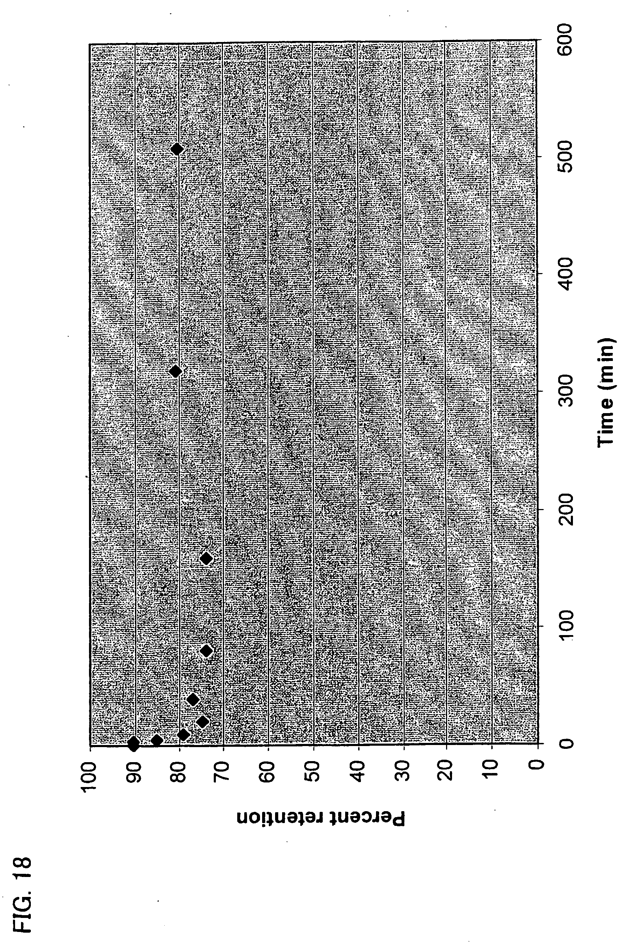

Quantification data for peptide 2DOS-2 retention (using data from the 300V scan) are shown in Table 9 and Figure 18:

**Table 9**

| Well | Minutes in 1 M NaCl / 10 mM tris-HCl (pH 8.0) | Untreated fluorescence (average) | Retained fluorescence | Percent retention |
|---|---|---|---|---|
| 1 | 0 | 1,000,613 | 904,211 | 90 |
| 2 | 2.5 | 1,000,613 | 902,082 | 90 |
| 3 | 5 | 1,000,613 | 847,767 | 85 |
| 4 | 10 | 1,000,613 | 792,427 | 79 |
| 5 | 20 | 1,000,613 | 749,522 | 75 |
| 6 | 40 | 1,000,613 | 769,659 | 77 |
| 7 | 80 | 1,000,613 | 740,227 | 74 |
| 8 | 160 | 1,000,613 | 739,600 | 74 |
| 9 | 320 | 1,000,613 | 805,530 | 81 |
| 10 | 510 | 1,000,613 | 803,741 | 80 |

The results show that retention of peptide 2DOS-2 on a hydrophobic polypropylene surface is stable for extended periods of time in 1 M NaCl / 10 mM tris-HCl (pH 8.0).

### Example 4

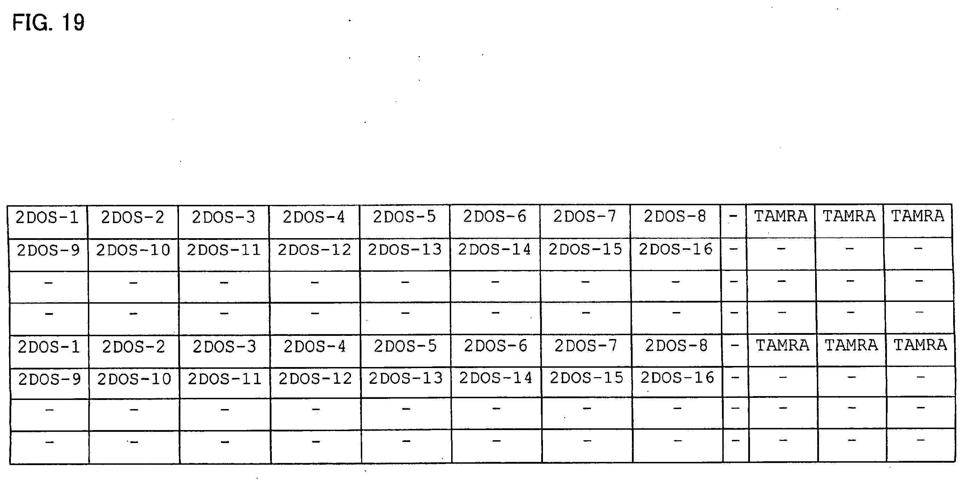

The retention of peptides 2DOS-1 to 2DOS-16 (see above) on polypropylene wells of different geometries was investigated:
10 mM tris-HCl (pH 8.0) in 50% (v/v) aqueous acetonitrile was used as the solvent for the peptides and for TAMRA.
1 µl aliquots of 10 µM peptides 2DOS-1 to 2DOS-16 and
10 µM TAMRA were pipetted into the wells of a Coster V-bottomed polypropylene microtitre plate and a Greiner flat-bottomed polypropylene microtitre plate according to the following scheme as shown in Figure 19:

The peptide samples were allowed to evaporate to dryness in the dark.

The peptide samples in the top two rows of the microtitre plates were then incubated for 15 minutes at room temperature in 250 µl of 1 M NaCl in 10 mM tris-HCl (pH 8.0). After incubation, the wash buffer was pipetted up and down eight times in the well before removing the supernatant.

The washed and entreated peptide samples were then resuspended in 50 µl of 10 mM tris-HCl (pH 8.0) in 50% (v/v) aqueous acetonitrile.

The microtitre plates were imaged at 200 µm resolution on a Typhoon Trio Plus variable mode imager (Amersham Biosciences) with the green (532 nm) laser and the 580 BP 30 filter at the PMT voltages indicated below and at normal sensitivity. The scan height was set at the platen and' the sample was pressed during scanning.

The fluorescence images were analysed using ImageQuant TL v2003.03 (Amersham Biosciences).

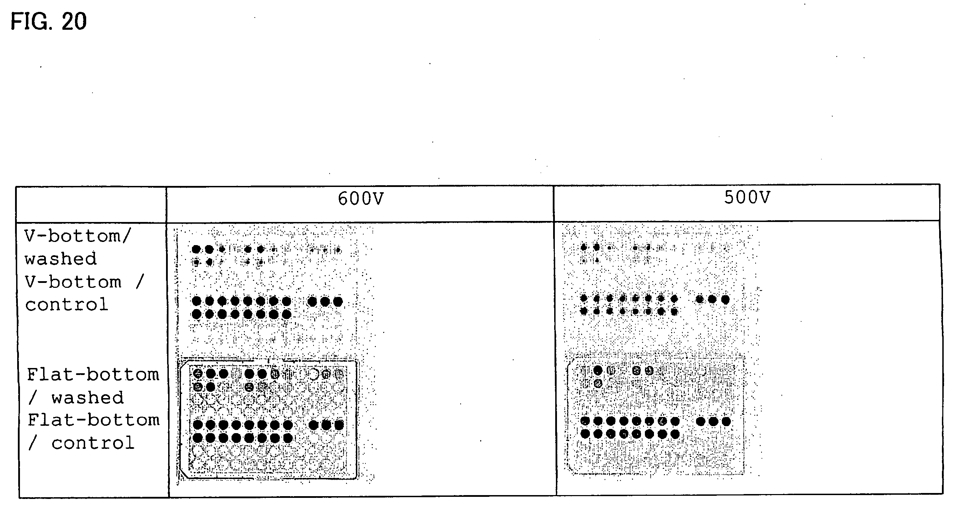

The fluorescence images of the plates scanned at PMT voltages of 600V and 500V are shown in Figure 20.

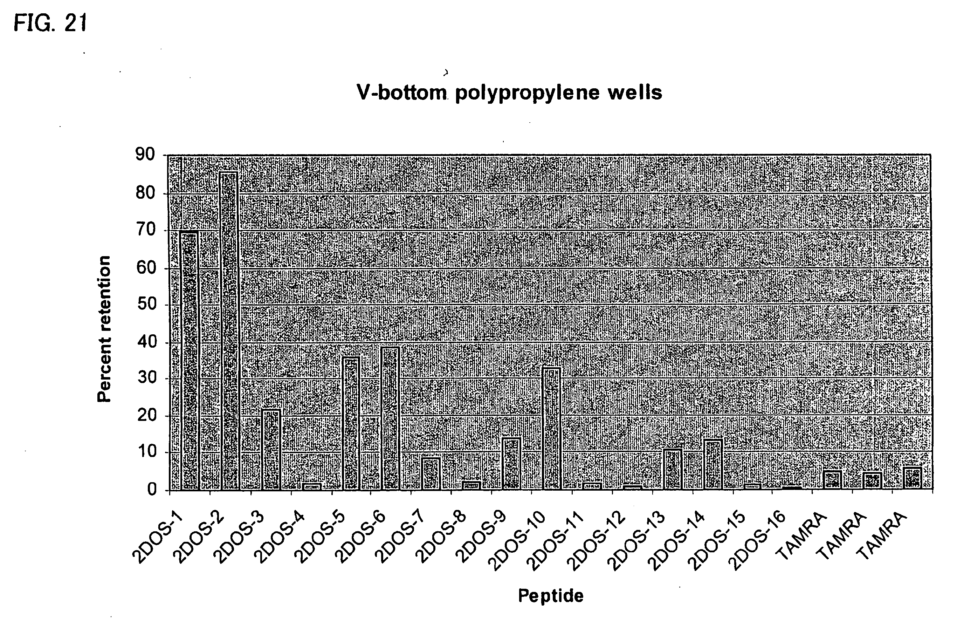

Quantification data for the V-bottom wells (using data from the 500V scan) are shown in Table 10 and Figure 21:

**Table 10**

| Peptide | Untreated fluorescence | Retained fluorescence | Percent retention |
|---|---|---|---|
| 2DOS-1 | 9,550,835 | 6,641,271 | 70 |
| 2DOS-2 | 9,495,183 | 8,127,309 | 86 |
| 2DOS-3. | 9,020,171 | 1,951,694 | 22 |
| 2DOS-4 | 8,265,596 | 159,080 | 2 |
| 2DOS-5 | 8,664,667 | 3,116,833 | 36 |
| 2DOS-6 | 9,141,290 | 3,527,116 | 39 |
| 2DOS-7 | 8,674,544 | 746,596 | 9 |
| 2DOS-8 | 10,130,734 | 202,943 | 2 |
| 2DOS-9 | 11,662,691 | 1,608,482 | 14 |
| 2DOS-10 | 8,445,090 | 2,773,876 | 33 |
| 2DOS-11 | 9,705,293 | 192,272 | 2 |
| 2DOS-12 | 6,777,661 | 52,365 | 1 |
| 2DOS-13 | 7,623,227 | 806,022 | 11 |
| 2DOS-14 | 7,641,246 | 1,018,179 | 13 |
| 2DOS-15 | 10,493,100 | 122,851 | 1 |
| 2DOS-16 | 9,782,527 | 44,420 | 0 |
| TAMRA | 12,610,993 | 640,684 | 5 |
| TAMRA | 15,392,751 | 686,863 | 4 |
| TAMRA | 17,471,866 | 998,320 | 6 |

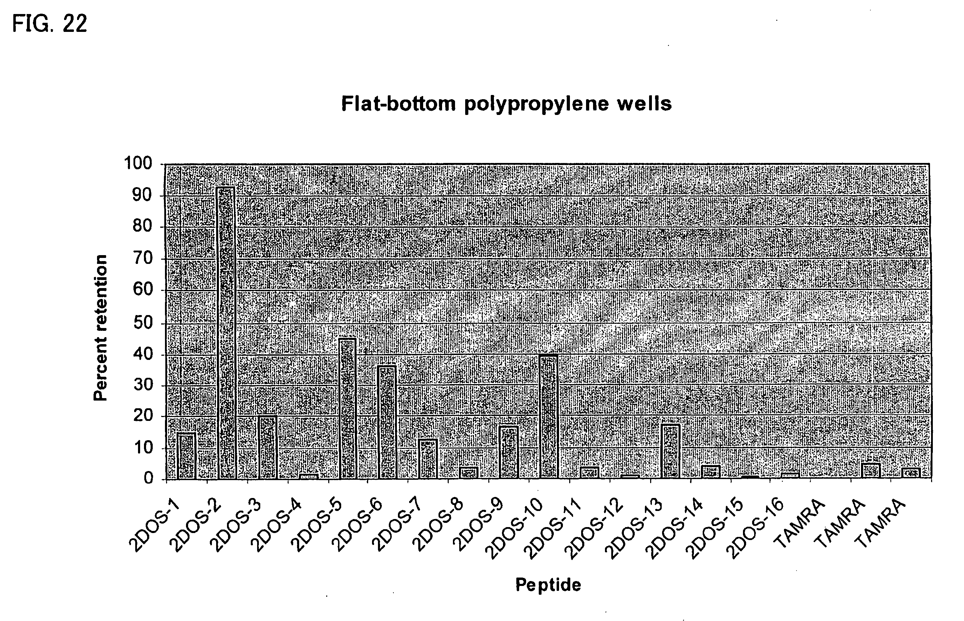

Quantification data for the flat-bottom wells (using data from the 500V scan) are shown in Table 11 and Figure 22:

**Table 11**

| Peptide | Untreated fluorescence | Retained fluorescence | Percent retention |
|---|---|---|---|
| 2DOS-1 | 14,949,396 | 2,157,124 | 14 |
| 2DOS-2 | 15,820,680 | 14,665,720 | 93 |
| 2DOS-3 | 14,152,875 | 2,836,015 | 20 |
| 2DOS-4 | 11,885,905 | 198,507 | 2 |
| 2DOS-5 | .14,337,629 | 6,492,170 | 45 |
| 2DOS-6 | 14,539,109 | 5,256,539 | 36 |
| 2DOS-7 | 10,189,473 | 1,303,195 | 13 |
| 2DOS-8 | 17,576,485 | 637,350 | 4 |
| 2DOS-9 | 13,456,698 | 2,148,849 | 16 |
| 2DOS-10 | 13,661,609 | 5,353,630 | 39 |
| 2DOS-11 | 13,960,552 | 476,318 | 3 |
| 2DOS-12 | 12,982,170 | 106,741 | 1 |
| 2DOS-13 | 12,935,739 | 2,162,164 | 17 |
| 2DOS-14 | 15,090,582 | 670,341 | 4 |
| 2DOS-15 | 15,290,885 | 104,523 | 1 |
| 2DOS-16 | 15,555,465 | 229,090 | 1 |
| TAMRA | 18,396,859 | 0 | 0 |
| TAMRA | 20,662,891 | 935,664 | 5 |
| TAMRA | 20,649,165 | 678,001 | 3 |

The results show that retention of peptides 2DOS-1 to 2DOS-16 on polypropylene wells of different geometries is comparable, indicating that retention is not dependent upon drying down in wells with a V-bottomed geometry.

### Example 5

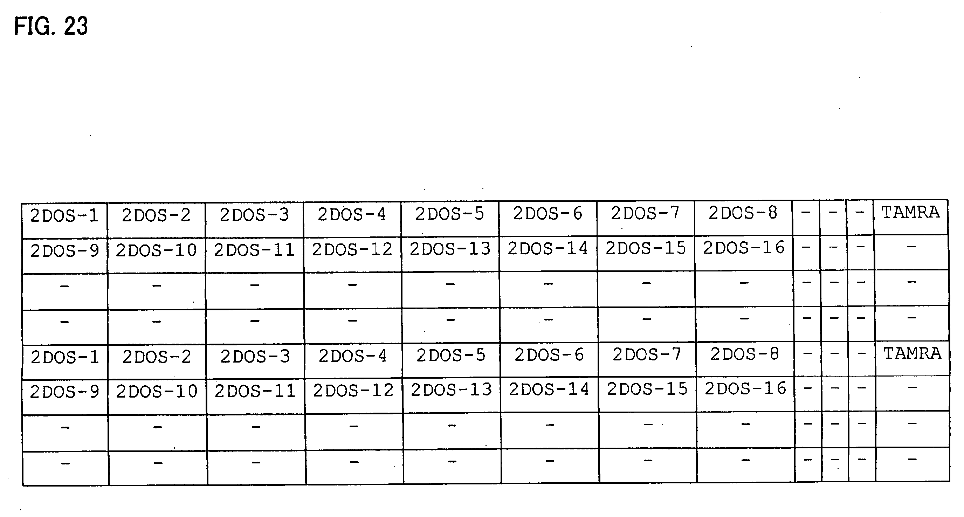

The retention of peptides 2DOS-1 to 2DOS-16 (see above) on polypropylene and polystyrene surfaces was compared:
5 mM tris-HCl (pH 8.0) in 75% (v/v) aqueous acetonitrile was used as the solvent for the peptides and for TAMRA.
20 µl aliquots of 5 µM peptides 2DOS-1 to 2DOS-16 and 5 µM TAMRA were pipetted into the wells of a Costar V-bottomed polypropylene microtitre plate, a Greiner V-bottomed polypropylene microtitre plate, and a Greiner V-bottomed polystyrene microtitre plate according to the scheme shown in Figure 23:

The peptide samples were allowed to evaporate to dryness in the dark.

The peptide samples in the top two rows of the microtitre plates were then incubated for 15 minutes at room temperature in 250 µl of 1 M NaCl in 10 mM tris-HCl (pH 8.0). After incubation, the wash buffer was pipetted up and down eight times in the well before removing the supernatant.

The washed and untreated peptide samples were then resuspended in 50 µl of 10 mM tris-HCl (pH 8.0) in 50% (v/v) aqueous acetonitrile.

The microtitre plates were imaged at 200 µm resolution on a Typhoon Trio Plus variable mode imager (Amersham Biosciences) with the green (532 nm) laser and the 580 BP 30 filter at the PMT voltages indicated below and at normal sensitivity. The scan height was set at +3 mm and the sample was pressed during scanning.

The fluorescence images were analysed using ImageQuant TL v2003.03 (Amersham Biosciences).

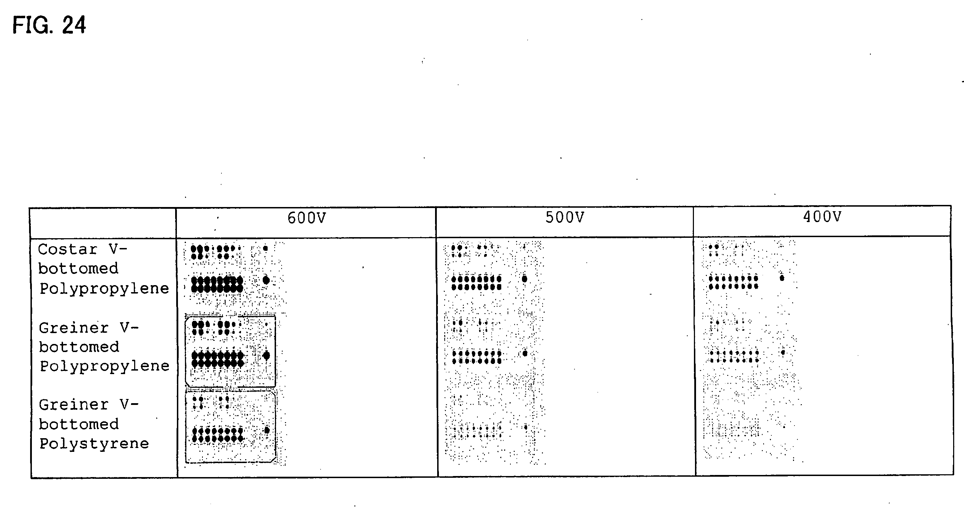

The fluorescence images of the slides scanned at PMT voltages of 600V, 500V, and 400V are shown in Figure 24:

Peptide samples in the upper half of the plates have been washed and peptide samples in the lower half of the plates are untreated.

Quantification data for the Costar polypropylene V-bottom wells (using data from the 400V scan) are given in Table 12:

**Table 12**

| Peptide | Untreated fluorescence | Retained fluorescence | Percent retention |
|---|---|---|---|
| 2DOS-1 | 6,458,364 | 3,366,403 | 52 |
| 2DOS-2 | 5,692,134 | 5,349,601 | 94 |
| 2DOS-3 | 5,660,618 | 673,940 | 12 |
| 2DOS-4 | 5,661,181 | 74,143 | 1 |
| 2DOS-5 | 5,331,763 | 1,651,850 | 31 |
| 2DOS-6 | 5,733,046 | 1,256,204 | 22 |
| 2DOS-7 | 5,139,578 | 326,328 | 6 |
| 2DOS-8 | 6,587,741 | 93,455 | 1 |
| 2DOS-9 | 7,102,251 | 1,184,648 | 17 |
| 2DOS-10 | 6,043,214 | 1,439,375 | 24 |
| 2DOS-11 | 5,327,435 | 102,040 | 2 |
| 2DOS-12 | 4,432,090 | 18,180 | 0 |
| 2DOS-13 | 4,886,617 | 387,738 | 8 |
| 2DOS-14 | 5,331,894 | 595,959 | 11 |
| 2DOS-15 | 6,488,130 | 50,584 | 1 |
| 2DOS-16 | 5,387,850 | 16,194 | 0 |
| TAMRA | 11,786,211 | 386,629 | 3 |

Quantification data for the Greiner polypropylene V-bottom wells (using data ,from the 400V scan) are given in Table 13:

**Table 13**

| Peptide | Untreated fluorescence | Retained fluorescence | Percent retention |
|---|---|---|---|
| 2DOS-1 | 3,328,9,60 | 1,235,460 | 37 |
| 2DOS-2 | 3,512,023 | 2,731,827 | 78 |
| 2DOS-3 | 3,583,352 | 342,889 | 10 |
| 2DOS-4 | 3,937,734 | 50,669 | 1 |
| 2DOS-5 | 3,817,250 | 1,048,978 | 27 |
| 2DOS-6 | 3,889,995 | 849,582 | 22 |
| 2DOS-7 | 3,751,280 | 205,430 | 5 |
| 2DOS-8 | 3,903,470 | 80,770 | 2 |
| 2DOS-9 | 3,621,913 | 439,252 | 12 |
| 2DOS-10 | 3,043,118 | 654,623 | 22 |
| 2DOS-11 | 3,510,896 | 86,818 | 2 |
| 2DOS-12 | 3,235,590 | 20,175 | 1 |
| 2DOS-13 | 3,635,229 | 378,287 | 10 |
| 2DOS-14 | 3,612,113 | 461,468 | 13 |
| 2DOS-15 | 4,771,265 | 65,142 | 1 |
| 2DOS-16 | 3,813,060 | 25,885 | 1 |
| TAMRA | 6,199,206 | 64,916 | 1 |

Quantification data for the Greiner polystyrene V-bottom wells (using data from the 400V scan) are given in Table 14:

**Table 14**

| Peptide | Untreated fluorescence | Retained fluorescence | Percent retention |
|---|---|---|---|
| 2DOS-1 | 640,145 | 225,686 | 35 |
| 2DOS-2 | 614,203 | 392,422 | 64 |
| 2DOS-3 | 744,747 | 37,388 | 5 |
| 2DOS-4 | 783,885 | 6,714 | 1 |
| 2DOS-5 | 745,029 | 171,500 | 23 |
| 2DOS-6 | 666,352 | 167,118 | 25 |
| 2DOS-7 | 706,200 | 15,766 | 2 |
| 2DOS-8 | 842,984 | 5,071 | 1 |
| 2DOS-9 | 626,304 | 62,023 | 10 |
| 2DOS-10 | 602,771 | 100,265 | 17 |
| 2DOS-11 | 675,269 | 7,365 | 1 |
| 2DOS-12 | 684,832 | 3,788 | 1 |
| 2DOS-13 | 708,162 | 62,590 | 9 |
| 2DOS-14 | 860,811 | 75,304 | 9 |
| 2DOS-15 | 868,334 | 5,116 | 1 |
| 2DOS-16 | 789,858 | 3,864 | 0 |
| TAMRA | 1,051,154 | 10,004 | 1 |

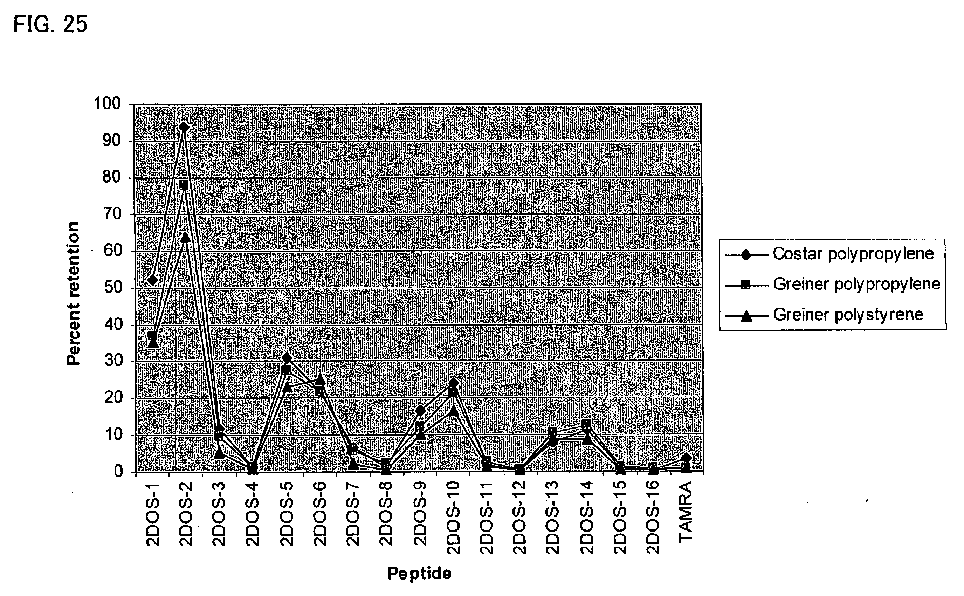

These data are shown graphically in Figure 25:

The results show that comparable peptide behaviour is seen on all three surfaces, demonstrating that retention is a sequence-specific property of the peptides rather than a property that is peculiar to one particular plastic surface.

### Example 6

Four peptides were synthesised that contain a 'surface-binding face' consisting of seven phenylalanyl residues. These peptides also contain a central region consisting of charged and uncharged residues and a variable penultimate residue. The variable penultimate residue was alanyl, seryl, cysteiyl, or nitropyridylthio activated cysteiyl.

An additional four TAMRA-labelled fluorescent peptides were also synthesised that contain an N-terminal TAMRA fluorophore attached to a glycyl residue that is attached to a variable C-terminal residue. The variable C-terminal residue was alanyl, seryl, cysteiyl, or nitropyridylthio activated cysteiyl.

A mixture of the 5-TAMRA and 6-TAMRA isomers as shown in Example 1 was used for the labelling.

The full set of eight peptides is shown in Tables 15 and 16:

**Table 15**

| Peptide | Sequence | Structure |
|---|---|---|
| SB-1 | N-Phe-Gly-Phe-Lys- | 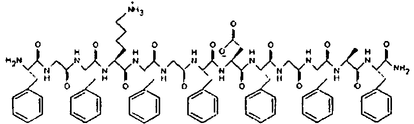 |
| | Phe-Gly-Phe-Asp- | |
| | Phe-Gly-Phe-Ala-Phe-C | |
| SB-2 | N-Phe-Gly-Phe-Lys- | 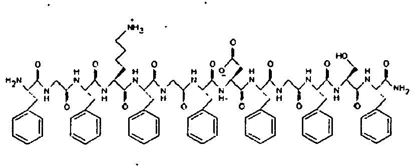 |
| | Phe-Gly-Phe-Asp- | |
| | Phe-Gly-Phe-Ser-Phe-C | |
| SB-3 | N-Phe-Gly-Phe-Lys- | 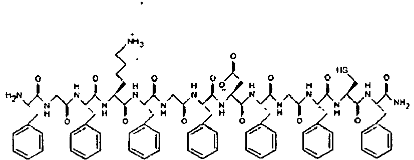 |
| | Phe-Gly-Phe-Asp- | |
| | Phe-Gly-Phe-Cys-Phe-C | |
| SB-4 | N-Phe-Gly-Phe-Lys- | 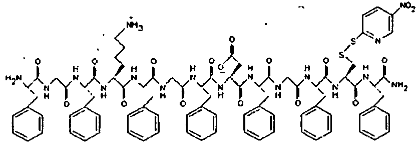 |
| | Phe-Gly-Phe-Asp- | |
| | Phe-Gly-Phe-CysSNP-Phe-C | |

**Table 16**

| Peptide | Sequence | Structure |
|---|---|---|
| TLSP-1 | N-TAMRA-Gly-Ala-C | 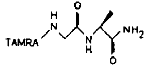 |
| TLSP-2 | N-TAMRA-Gly-Ser-C | 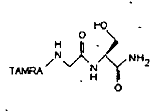 |
| TLSP-3 | N-TAMRA-Gly-Cys-C | 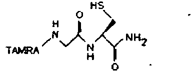 |
| TLSP-4 | N-TAMRA-Gly-CysSNP-C | 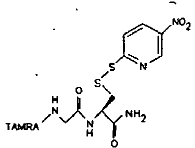 |

The peptides SB-1 to SB-4 and TLSP-1 to TLSP-4 were used in order to investigate dimer formation.

Two different protocols were used. In the 'liquid phase' protocol, The SB peptides were dried down onto a polypropylene surface. The TLSP peptides were then added in aqueous solution prior to washing, the wells and assaying for retained fluorescent material.

In the 'co-drying' protocol, The SB peptides were mixed with the TLSP peptides and both were then dried down together onto a polypropylene surface prior to washing the wells and assaying for retained fluorescent material.

A further protocol in which the SB peptides are mixed with the TLSP peptides in aqueous solution and allowed to react to produce peptide dimers which are then dried down onto a polypropylene surface could easily be achieve by one skilled in the art.

In the 'liquid phase' protocol, 50 µl of 10 µM peptides SB-1 to SB-4 in 1 mM NaH₂PO₄ in 50% (v/v) aqueous acetonitrile were added the wells of a Costar V-bottomed micr.otitre plate according to the scheme shown in Table 17:

**Table 17**

| | | | | | | | | | | | |
|---|---|---|---|---|---|---|---|---|---|---|---|
| SB-1 | SB-2 | SB-3 | SB-4 | - | - | - | - | - | - | - | - |
| SB-1 | SB-2 | SB-3 | SB-4 | - | - | - | - | - | - | - | - |
| SB-1 | SB-2 | SB-3 | SB-4 | - | - | - | - | - | - | - | - |
| SB-1 | SB-2 | SB-3 | SB-4 | - | - | - | - | - | - | - | - |
| SB-1 | SB-2 | SB-3 | SB-4 | - | - | - | - | - | - | - | - |
| - | - | - | - | - | - | - | - | - | - | - | - |

The samples were dried down overnight in the dark. 50 µl of 100 µM peptides TLSP-1 to TLSP-4 in 10 mM NaH₂PO₄ were then added to the wells according to the scheme shown in Table 18:

**Table 18**

| | | | | | | | | | | | |
|---|---|---|---|---|---|---|---|---|---|---|---|
| TLSP-1 | TLSP-1 | TLSP-1 | TLSP-1 | TLSP-1 | - | - | - | - | - | - | - |
| TLSP-2 | TLSP-2 | TLSP-2 | TLSP-2 | TLSP-2 | - | - | - | - | - | - | - |
| TLSP-3 | TLSP-3 | TLSP-3 | TLSP-3 | TLSP-3 | - | - | - | - | - | - | -, |
| TLSP-4 | TLSP-4 | TLSP-4 | TLSP-4 | TLSP-4 | - | - | - | - | - | - | - |
| - | - | - | - | - | - | - | - | - | - | - | - |
| - | - | - | - | - | - | - | - | - | - | - | - |
| - | - | - | - | - | - | - | - | - | - | - | - |
| - | - | - | - | - | - | - | - | - | - | - | - |

The samples were incubated at room temperature for one hour in the dark. The supernatants were removed and the wells were washed twice with 200 µl of 1 M NaCl in 10 mM tris-HCl (pH 8.0). 50 µl of 10 mM tris-HCl (pH 8.0) in 50% (v/v) aqueous acetonitrile was finally added to the wells.

The microtitre plate was imaged at 200 µm resolution on a Typhoon Trio Plus variable mode imager (Amersham Biosciences) with the green (532 nm) laser and the 580 BP 30 filter at a PMT voltage of 500 V and at normal sensitivity. The scan height was set art +3 mm and the sample was pressed during scanning. The fluorescence image was analysed using ImageQuant TL v2003.03 (Amersham Biosciences).

In the 'co-drying' protocol, 50 µl of 10 µM peptides SB-1 to SB-4 in 1 mM NaH₂PO₄ in 50% (v/v) aqueous acetonitrile were added to the wells of a Costar V-bottomed microtitre plate according to the scheme shown in Table 19:

**Table 19**

| | | | | | | | | | | | |
|---|---|---|---|---|---|---|---|---|---|---|---|
| SB-1 | SB-2 | SB-3 | SB-4 | - | - | - | - | - | - | - | - |
| SB-1 | SB-2 | SB-3 | SB-4 | - | - | - | - | - | - | - | - |
| SB-1 | SB-2 | SB-3 | SB-4 | - | - | - | - | - | - | - | - |
| SB-1 | SB-2 | SB-3 | SB-4 | - | - | - | - | - | - | - | - |
| SB-1 | SB-2 | SB-3 | SB-4 | - | - | - | - | - | - | - | - |
| - | - | - | - | - | - | - | - | - | - | - | - |
| - | - | - | - | - | - | - | - | - | - | - | - |
| - | - | - | - | - | - | - | - | - | - | - | - |

50 µl of 100 µM peptides TLSP-1 to TLSP-4 in 1 mM NaH₂PO₄ in 50% (v/v) aqueous acetonitrile were then added to the wells according to the scheme shown in Table 20:

**Table 20**

| | | | | | | | | | | | |
|---|---|---|---|---|---|---|---|---|---|---|---|
| TLSP-1 | TLSP-1 | TLSP-1 | TLSP-1 | TLSP-1 | - | - | - | - | - | - | - |
| TLSP-2 | TLSP-2 | TLSP-2 | TLSP-2 | TLSP-2 | - | - | - | - | - | - | - |
| TLSP-3 | TLSP-3 | TLSP-3 | TLSP-3 | TLSP-3 | - | - | - | - | - | - | - |
| TLSP-4 | TLSP-4 | TLSP-4 | TLSP-4 | TLSP-4 | - | - | - | - | - | - | - |
| - | - | - | - | - | - | - | - | - | - | - | - |
| - | - | - | - | - | - | - | - | - | - | - | - |
| - | - | - | - | - | - | - | - | - | - | - | - |
| - | - | - | - | - | - | - | - | - | - | - | - |

The samples were dried down overnight in the dark.

The wells were then washed twice with 200 µl of 1 M NaCl in 10 mM tris-HCl (pH 8.0). 50 µl of 10 mM tris-HCl (pH 8.0) in 50% (v/v) aqueous acetonitrile was finally added to the wells.

The microtitre plate was imaged at 200 µm resolution on a Typhoon Trio Plus variable mode imager (Amersham Biosciences) with the green (532 nm) laser and the 580 BP 30 filter at a PMT voltage of 500 V and at normal sensitivity. The scan height was set at +3 mm and the sample was pressed during scanning. The fluorescence image was analysed using ImageQuant TL v2003.03 (Amersham Biosciences).

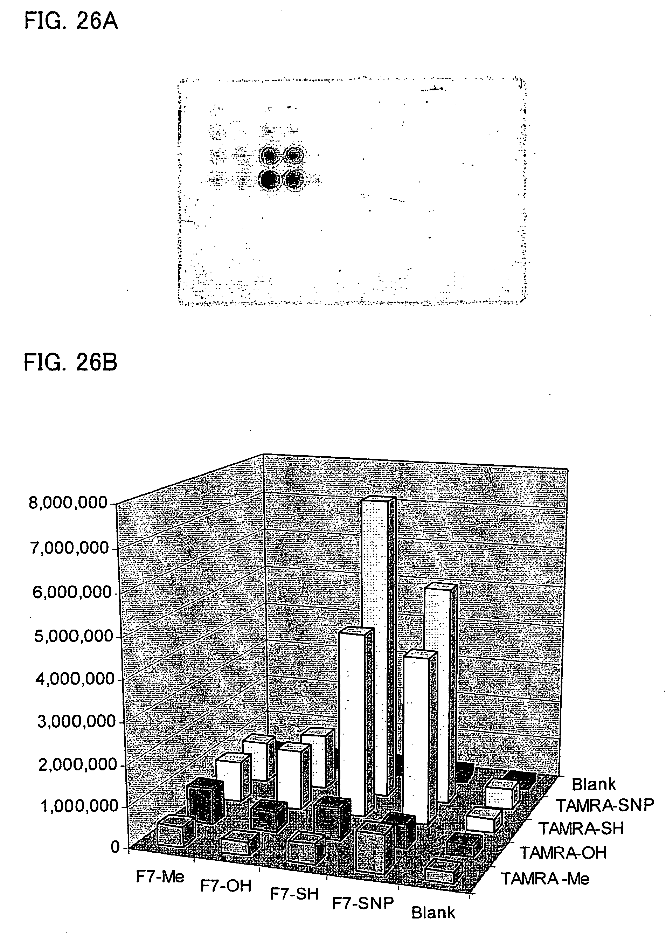

The fluorescence image for the microtitre plate from the experiment using the 'liquid phase' protocol is shown in Figure 26A.

The fluorescence data for the 'liquid phase' protocol are given in Table 21:

**Table 21**

| | SB-1 (F7-Me) | SB-2 (F7-OH) | SB-3 (F7-SH) | SB-4 (F7-SNP) | Blank |
|---|---|---|---|---|---|
| TLSP-1-(TAMRA-Me) | 505,542 | 328,552 | 494,464 | 940,493 | 250,236 |
| TLSP-2 (TAMRA-OH) | 875,810 | 495,642 | 790,731 | 574,079 | 279,409 |
| TLSP-3 (TAMRA-SH | 1,024,752 | 1,449,785 | 4,531,849 | 4,101,860 | 341,250 |
| TLSP-4 (TAMRA-SNP) | 1,021,924 | 1,357,602 | 7,434,703 | 5,37,8,576 | 522,053 |
| Blank | 266,620 | 246,461 | 285,687 | 265,120 | 203,597 |

The results are shown graphically in Figure 26B:

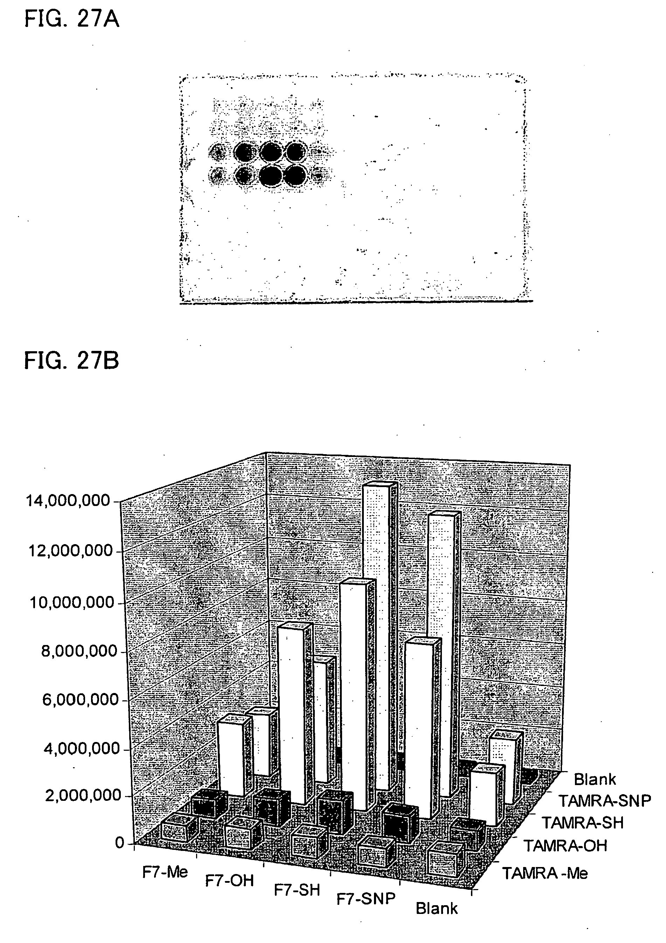

The fluorescence image for the microtitre plate from the experiment using the 'co-drying' protocol is shown in Figure 27A.

The fluorescence data for the 'co-drying' protocol are given in the following Table 22:

**Table 22**

| | SB-1 (F7-Me) | SB-2 (F7-OH) | SB-3 (F7-SH) | SB-4 (F7-SNP) | Blank |
|---|---|---|---|---|---|
| TLSP-1 (TAMRA-Me) | 719,315 | 906,087 | 919,079 | 807,652 | 970,904 |
| TLSP-2 (TAMRA-OH) | 816,019 | 1,165,325 | 1,458,222 | 1,163,929 | 892,135 |
| TLSP-3 (TAMRA-SH | 3,301,896 | 7,778,287 | 9,886,083 | 7,559,276 | 2,317,125 |
| TLSP-4 (TAMRA-SNP) | 2,862,439 | 5,555,447 | 13,506,288 | 12,389,014 | 2,876,776 |
| Blank | 258,401 | 295,964 | 366,184 | 378,912 | 231,826 |

The results are shown graphically in Figure 27B:

The yield of dimer is assayed by the retention of fluorescently labelled peptide which is conditional upon the presence of an unlabelled peptide that can bind to both the polypropylene surface and to the fluorescently labelled peptide.

For the 'liquid phase protocol' dimer yields are lower than for the 'co-drying' protocol but the chemical specificity for dimer formation is better. Maximal dimer formation is seen when the surface 'peptide possesses a free thiol group and the solution peptide possesses an S-nitropyridyl activated thiol group. Dimer formation is also observed when the surface peptide possesses an S-nitropyridyl activated thiol group and the solution peptide also possesses an S-nitropyridyl activated thiol group; when the surface peptide possesses a free thiol group and the solution peptide also possesses a free thiol group; and when the surface peptide possesses an S-nitropyridyl activated thiol group and the solution peptide possesses a free thiol group.

Free thiol coupling to free thiols may be due to simple aerobic oxidation, forming disulfide bonds. S-nitropyridyl activated thiol coupling to S-nitropyridyl activated thiols may be a result of incomplete thiol activation, leaving some free thiols able to react with the remaining S-nitropyridyl activated thiols, or some other mechanism.

Results, for the 'co-drying', protocol, mirror those described above. Dimer yields are generally higher with this method but non-specific binding is also higher. In particular, some reactivity is observed between a hydroxylated peptide attached to the surface and solution peptides containing both free thiol groups and S-nitropyridyl activated thiol groups.

### Example 7

In this example, peptide dimers are fabricated on a planar plastic surface using a Piezorray (PerkinElmer LAS) non-contact dispenser. The Piezorray (PerkinElmer LAS) is specifically designed for pipetting nanolitre volumes to dense arrays. Liquid volumes are controlled by a piezoelectric tip. The Piezorray system contains a source plate holder, an ultrasonic washbowl, a computer and monitor, and a bottle for system liquid.

Polypropylene sheet was obtained from SBA plastics (http://www.sba.co.uk/, Propylex Natural Polypropylene Sheet 2440 x 1220 x 1 mm) and was wiped with 50% (v/v) aqueous acetonitrile prior to use.

Six 10x10 arrays of 5nl of 100µM peptides SB-1 and SB-3 in 1mM NaH₂PO4 in 50% (v/v) aqueous acetonitrile or solvent control were dispensed to 1 mm polypropylene sheet cut to 3"x1" using the Piezorray system according to the scheme shown in Table 23.

**Table 23**

| |
|---|
| 1 mM NaH₂PO₄ in 50% (v/v) aqueous acetonitrile |
| 1 mM NaH₂PO₄ in 50% (v/v) aqueous acetonitrile |
| Peptide SB-1 |
| Peptide SB-1 |
| Peptide SB-3 |
| Peptide SB-3 |

Six 10x10 arrays, of 5 nl of 100 µM peptide TLSP-4 in either 1 mM NaH₂PO₄ in 50% (v/v) aqueous acetonitrile or in 1 mM NaH₂PO₄ in 50% (v/v) aqueous acetonitrile and 10% (v/v) glycerol were then dispensed over the previous spots using the Piezorray system according to the scheme shown in Table 24:

**Table 24**

| |
|---|
| Peptide TLSP-4 |
| Peptide TLSP-4 in 10% glycerol |
| Peptide TLSP-4 |
| Peptide TLSP-4 in 10% glycerol |
| Peptide TLSP-4 |
| Peptide TLSP-4 in 10% glycerol |

The samples were incubated at room temperature for 30 minutes in the dark. The slide was then washed in 100 ml of 50 mM NaCl in 10 mM tris-HCl (pH 8.0) followed by running tap water over the slide for one minute.

The microtitre plate was imaged at 10 µm resolution on a Typhoon Trio Plus variable mode imager (Amersham Biosciences) with the green (532 nm) laser and the 580 BP 30 filter at a PMT voltage of 400 V and at normal sensitivity. The scan height was set at the platen and the sample was pressed during scanning: The fluorescence image was analysed using ImageQuant TL v2003.03 (Amersham Biosciences).

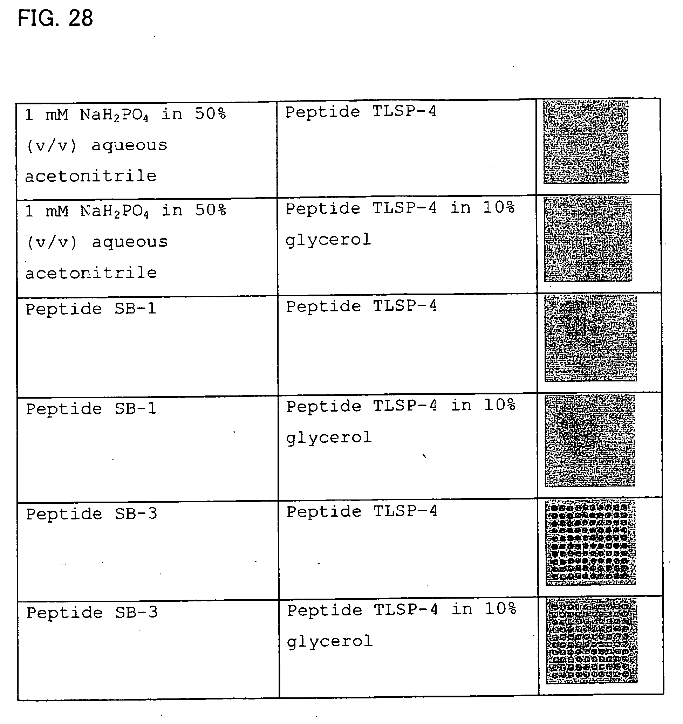

She fluorescence image for the polypropylene slide is shown in Figure 28:

The yield of dimer is assayed by the detention of fluorescently labelled peptide that is conditional upon the presence of an unlabelled peptide that can bind to both the polypropylene surface and to the fluorescently labelled peptide.

Dimer formation is therefore seen when the surface peptide possesses a free thiol group and the solution peptide possesses an S-nitropyridyl activated thiol group. The simple protocol (without glycerol to prevent evaporation) gives a higher yield of dimer.

### Example 8

In this example, twenty 256-element microarrays of dimers comprising peptides L1-P1-1 to 16 and L1-P2-1 to 16 were fabricated in parallel.

1 mm polypropylene sheet was cut to 136 mm x 80mm, lightly abraded with glass paper, and wiped with 50% (v/v) aqueous acetonitrile prior to use.

18x 6 nl aliquots of P1 peptides were arrayed down the columns of the polypropylene slide at a spacing of 0.72 mm as indicated in Table 25, using the Piezorray system (PerkinElmer LAS).

**Table 25**

| Column number | P1 peptide | Solvent |
|---|---|---|
| 1 | 2 µM P1-5 | 90% (v/v) DMSO, 1 mM tris-HCl (pH 8.0) |
| 2 | 20 µM L1-P1-1 | 90% (v/v) DMSO, 1 mM tris-HCl (pH 8.0), 2 mM TCEP |
| 3 | 20 µM L1-P1-2 | 90% (v/v) DMSO, 1 mM tris-HCl (pH 8.0), 2 mM TCEP |
| 4 | 20 µM L1-P1-3 | 90% (v/v) DMSO, 1 mM tris-HCl (pH 8.0), 2 mM TCEP |
| 5 | 20 µM L1-P1-4 | 90% (v/v) DMSO, 1 mM tris-HCl (pH.8.0), 2 mM TCEP |
| 6 | 20 µM L1-P1-5 | 90% (v/v) DMSO, 1 mM tris-HCl (pH 8.0), 2 mM TCEP |
| 7 | 20 µM L1-P1-6 | 90% (v/v) DMSO, 1 mM tris-HCl (pH 8.0), 2 mM TCEP |
| 8 | 20 µM L1-P1-7 | 90% (v/v) DMSO, 1 mM tris-HCl (pH 8.0), 2 mM TCEP |
| 9 | 20 µM L1-P1-8 | 90% (v/v) DMSO, 1 mM tris-HCl (pH 8.0), 2 mM TCEP |
| 10 | 20 µM L1-P1-9 | 90% (v/v) DMSO, 1 mM tris-HCl (pH 8.0); 2 mM TCEP |
| 11 | 20 µM L1-P1-10 | 90% (v/v) DMSO, 1 mM tris-HCl (pH 8.0), 2 mM TCEP |
| 12 | 20 µM L1-P1-11 | 90% (v/v) DMSO, 1 mM tris-HCl (pH 8.0), 2 mM TCEP |
| 13 | 20 µM L1-P1-12 | 90% (v/v) DMSO, 1 mM tris-HCl (pH 8.0), 2 mM TCEP |
| 14 | 20 µM L1-B1-13 | 90% (v/v) DMSO, 1 mM tris-HCl (pH 8.0), 2 mM TCEP |
| 15 | 20 µM L1-P1-14 | 90% (v/v) DMSO, 1 mM tris-HCl (pH 8.0), 2 mM TCEP |
| 16 | 20 µM L1-P1-15 | 90% (v/v) DMSO, 1 mM tris-HCl (pH 8.0), 2 mM TCEP |
| 17 | 20 µM L1-P1-16 | 90% (v/v) DMSO, 1 mM tris-HCl (pH 8.0), 2 mM TCEP |
| 18 | 2 µM P1-5 | 90% (v/v) DMSO, 1 mM tris-HCl (pH 8.0) |

| | | |
|---|---|---|
| DMSO = dimethyl sulfoxide TCEP = tris (2-carboxyethyl) phosphine | | |

### Sequence of P1 peptides:

| | |
|---|---|
| P1-5 | TAMRA-Phe-Gly-Phe-Gly-Phe-Gly-Phe-Gly-Phe-Gly-Phe-Ser-Phe-Ala-Phe-Asp-Phe-Gly-Phe |
| L1-P1-1 | Phe-Gly-Phe-Gly-Phe-Gly-Phe-Gly-Phe-Gly-Phe-Gly-Phe-Gly-Phe-Cys-Phe-DAB-Phe-DAB-Phe-Gly-Phe |
| L1-P1-2 | Phe-Gly-Phe-Gly-Phe-Gly-Phe-Gly-Phe-Gly-Phe-Gly-Phe-Gly-Phe-Cys-Phe-DAB-Phe-Hse-Phe-Gly-Phe |
| L1-P1-3 | Phe-Gly-Phe-Gly-Phe-Gly-Phe-Gly-Phe-Gly-Phe-Gly-Phe-Gly-Phe-Cys-Phe-DAB-Phe-Abu-Phe-Gly-Phe |
| L1-P1-4 | Phe-Gly-Phe-Gly-Phe-Gly-Phe-Gly-Phe-Gly-Phe-Gly-Phe-Gly-Phe-Cys-Phe-DAB-Phe-Asp-Phe-Gly-Phe |
| L1-P1-5 | Phe-Gly-Phe-Gly-Phe-Gly-Phe-Gly-Phe-Gly-Phe-Gly-Phe-Gly-Phe-Cys-Phe-Hse-Phe-DAB-Phe-Gly-Phe |
| L1-P1-6 | Phe-Gly-Phe-Gly-Phe-Gly-Phe-Gly-Phe-Gly-Phe-Gly-Phe-Gly-Phe-Cys-Phe-Hse-Phe-Hse-Phe-Gly-Phe |
| L1-P1-7 | Phe-Gly-Phe-Gly-Phe-Gly-Phe-Gly-Phe-Gly-Phe-Gly-Phe-Gly-Phe-Cys-Phe-Hse-Phe-Abu-Phe-Gly-Phe |
| L1-P1-8 | Phe-Gly-Phe-Gly-Phe-Gly-Phe-Gly-Phe-Gly-Phe-Gly-Phe-Gly-Phe-Cys-Phe-Hse-Phe-Asp-Phe-Gly-Phe |
| L1-P1-9 | Phe-Gly-Phe-Gly-Phe-Gly-Phe-Gly-Phe-Gly-Phe-Gly-Phe-Gly-Phe-Cys-Phe-Abu-Phe-DAB-Phe-Gly-Phe |
| L1-P1-1 | 0 Phe-Gly-Phe-Gly-Phe-Gly-Phe-Gly-Phe-Gly-Phe-Gly-Phe-Gly-Phe-Cys-Phe-Abu-Phe-Hse-Phe-Gly-Phe |
| L1-P1-11 | Phe-Gly-Phe-Gly-Phe-Gly-Phe-Gly-Phe-Gly-Phe-Gly-Phe-Gly-Phe-Cys-Phe-Abu-Phe-Abu-Phe-Gly-Phe |
| L1-P1-12 | Phe-Gly-Phe-Gly-Phe-Gly-Phe-Gly-Phe-Gly-Phe-Gly-Phe-Gly-Phe-Cys-Phe-Abu-Phe-Asp-Phe-Gly-Phe |
| L1-P1-13 | Phe-Gly-Phe-Gly-Phe-Gly-Phe-Gly-Phe-Gly-Phe-Gly-Phe-Gly-Phe-Cys-Phe-Asp-Phe-DAB-Phe-Gly-Phe |
| L1-P1-14 | Phe-Gly-Phe-Gly-Phe-Gly-Phe-Gly-Phe-Gly-Phe-Gly-Phe-Gly-Phe-Cys-Phe-Asp-Phe-Hse-Phe-Gly-Phe |
| L1-P1-15 | Phe-Gly-Phe-Gly-Phe-Gly-Phe-Gly-Phe-Gly-Phe-Gly-Phe-Gly-Phe-Cys-Phe-Asp-Phe-Abu-Phe-Gly-Phe |
| L1-P1-16 | Phe-Gly-Phe-Gly-Phe-Gly-Phe-Gly-Phe-Gly-Phe-Gly-Phe-Gly-Phe-Cys-Phe-Asp-Phe-Asp-Phe-Gly-Phe |

After sample evaporation, 18x 12 nl aliquots of L1-P2 peptides in 90% (v/v) DMSO, 1 mM tris-HCl (pH 8.0) were arrayed along the rows of the polypropylene slide at a spacing of 0.72 mm as indicated in Table 26, using the Piezorray system (PerkinElmer LAS).

**Table 26**

| Row number | Unlabelled P2 peptide | Labelled P2 peptides |
|---|---|---|
| 1 | - | 25 µM combined concentration of L1-P2-17/18/19/20 mixture |
| 2 | 50 µM L1-P2-1 | 25 µM combined concentration of L1-P2-17/18/19/20 mixture |
| 3 | 50 µM L1-P2-2 | 25 µM combined concentration of L1-P2-17/18/19/20 mixture |
| 4 | 50 µM L1-P2-3 | 25 µM combined concentration of L1-P2-17/18/19/20 mixture |
| 5 | 50 µM L1-P2-4 | 25 µM combined concentration of L1-P2-17/18/19/20 mixture |
| 6 | 50 µM L1-P2-5 | 25 µM combined concentration of L1-P2-17/18/19/20 mixture |
| 7 | 50 µM L1-P2-6 | 25 µM combined concentration of L1-P2-17/18/19/20 mixture |
| 8 | 50 µM L1-P2-7 | 25 µM combined concentration of L1-P2-17/18/19/20 mixture |
| 9 | 50 µM L1-P2-8 | 25 µM combined concentration of L1-P2-17/18/19/20 mixture |
| 10 | 50 µM L1-P2-9 | 25 µM combined concentration of L1-P2-17/18/19/20 mixture |
| 11 | 50 µM L1-P2-10 | 25 µM combined concentration of L1-P2-17/18/19/20 mixture |
| 12 | 50 µM L1-P2-11 | 25 µM combined concentration of L1-P2-17/18/19/20 mixture |
| 13 | 50 µM L1-P2-12 | 25 µM combined concentration of L1-P2-17/18/19/20 mixture |
| 14 | 50 µM L1-P2-13 | 25 µM combined concentration of L1-P2-17/18/19/20 mixture |
| 15 | 50 µM L1-P2-14 | 25 µM combined concentration of L1-P2-17/18/19/20 mixture |
| 16 | 50 µM L1-P2-15 | 25 µM combined concentration of L1-P2-17/18/19/20 mixture |
| 17 | 50 µM L1-P2-16 | 25 µM combined concentration of L1-P2-17/18/19/20 mixture |
| 18 | - | 25 µM combined concentration of L1-P2-17/18/19/20 mixture |

### Sequence of P2 peptides:

| | |
|---|---|
| L1-P2-1 | CysSTP-DAB-Phe-DAB-Phe-Gly-Phe |
| L1-P2-2 | CysSTP-DAB-Phe-Hse-Phe-Gly-Phe |
| L1-P2-3 | CysSTP-DAB-Phe-Abu-Phe-Gly-Phe |
| L1-P2-4 | CysSTP-DAB-Phe-Asp-Phe-Gly-Phe |
| L1-P2-5 | CysSTP-Hse-Phe-DAB-Phe-Gly-Phe |
| L1-P2-6 | CysSTP-Hse-Phe-Hse-Phe-Gly-Phe |
| L1-P2-7 | CysSTP-Hse-Phe-Abu-Phe-Gly-Phe |
| L1-P2-8 | CysSTP-Hse-Phe-Asp-Phe-Gly-Phe |
| L1-P2-9 | CysSTP-Abu-Phe-DAB-Phe-Gly-Phe |
| L1-P2-10 | CysSTP-Abu-Phe-Hse-Phe-Gly-Phe |
| L1-P2-11 | CysSTP-Abu-Phe-Abu-Phe-Gly-Phe |
| L1-P2-12 | CysSTP-Abu-Phe-Asp-Phe-Gly-Phe |
| L1-P2-13 | CysSTP-Asp-Phe-DAB-Phe-Gly-Phe |
| L1-P2-14 | CysSTP-Asp-Phe-Hse-Phe-Gly-Phe |
| L1-P2-15 | CysSTP-Asp-Phe-Abu-Phe-Gly-Phe |
| L1-P2-16 | CysSTP-Asp-Phe-Asp-Phe-Gly-Phe |
| L1-P2-17 | TAMRA-CysSTP-DAB-Phe-DAB-Phe-Gly-Phe |
| L1-P2-18 | TAMRA-CysSTP-Hse-Phe-Hse-Phe-Gly-Phe |
| L1-P2-19 | TAMRA-GysSTP-Abu-Phe-Abu-Phe-Gly-Phe |
| L1-P2-20 | TAMRA-CysSTP-Asp-Phe-Asp-Phe-Gly-Phe |

After sample evaporation, the slide was washed for 10 minutes in 50 ml of 10 mM tris-HCl (pH 8.0) containing 0.1% (v/v) Tween-20.

The slide was imaged at 10 µm resolution on a Typhoon Trio Plus variable mode imager (Amersham Biosciences) with the green (532 nm) laser and the 580 BP 30 filter at a PMT voltage of 500V and at normal sensitivity. The scan height was set at the platen. The fluorescence image was analysed using ImageQuant TL v2003.03 (Amersham Biosciences).

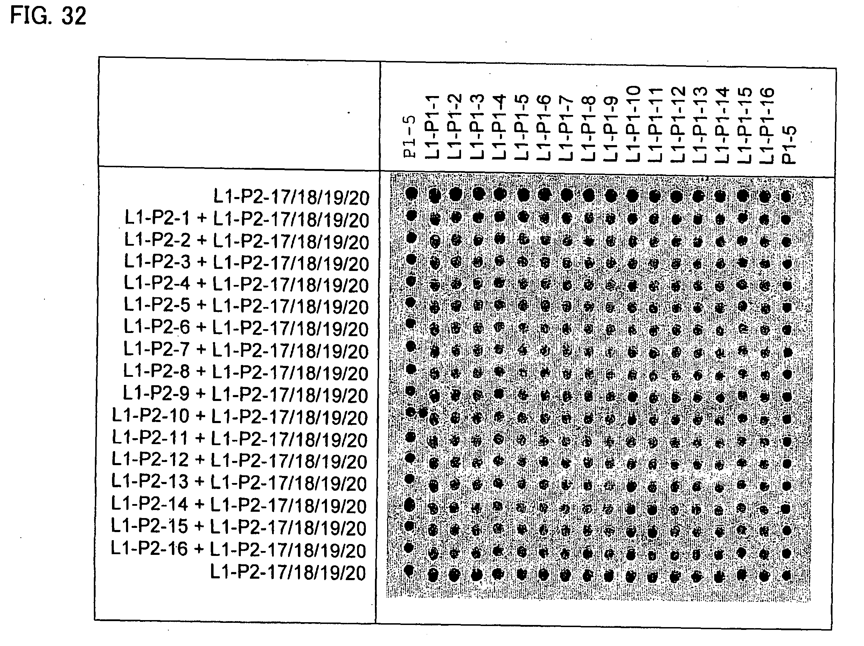

The fluorescence image for one 18x18 array of dimer and control spots is shown in Figure 32.

Fluorescent signal is observable for each L1-P1 peptide column dispensed to the array. This indicates that each of the L1-P1 peptides has been successfully dispensed, and is capable of dimer formation. The fluorescent signal is also observable for each L1-P2 peptide row dispensed to the array. This indicates that each of the L1-P2 peptides has been successfully dispensed, and is capable of dimer formation.

The dimer fluorescence is greater for the samples with only TAMRA-labelled P2 peptides compared to the dimer fluorescence for the 16x16 array fabricated with both unlabelled P2 peptides and TAMRA-labelled P2 peptides competing for the L1-P1 peptide thiol groups. This indicates that all of the L1-P2 peptides have successfully competed with their TAMRA-labelled counterparts and have therefore successfully formed peptide dimers between all sixteen L1- P1 peptides and all sixteen L1-P2 peptides.

## Claims

1. An array of peptide dimer ligand-binding capture agents for capturing an analyte in a sample wherein:
each of said ligand-binding capture agents comprises first and second peptide chains being formed as a dimer by means of a covalent link therebetween, wherein said covalent link has been formed by reaction of first and second thiol groups on said first and second peptides,
each of said first and second peptide chains comprises a chain of between 5 and 25 amino acids wherein each amino acid is a D-enantiomer or an L-enantiomer and has been selected from a set of less than 6 amino acids,
each of said first and second peptide chains have different amino acid sequences,
said first peptide is immobilised via an attachment moiety to a substrate, said attachment moiety being selected from the group consisting of a hydrophobic moiety and a moiety forming a covalent bond with the substrate and wherein
the first and second peptide chains are combinatorially-varied such that all possible combinations of amino acids present in the said set are present in the array and said array comprises discrete spatially addressable locations and each location comprises a different capture agent.

2. An array as claimed in claim 1 wherein each amino acid has been selected from a set of 4 amino acids.

3. An array as claimed in any preceding claim wherein the ligand comprises a eukaryotic cell, a prokaryotic cell, a virus, a bacteriophage, a prion, a spore, a pollen grain, an allergen, a nucleic acid, a protein, a peptide, a carbohydrate, a lipid, an inorganic compound or an organic compound.

## Patentansprüche

1. Ein Array aus Peptid-Dimer-Ligand-Bindenden-Einfangreagenzien zum Einfangen eines Analyten in einer Probe, wobei:
jedes der Ligand-Bindenden-Einfangreagenzien erste und zweite Peptidketten aufweist, als Dimer mittels einer kovalenten Bindung dazwischen gebildet, wobei die kovalente Bindung durch eine Reaktion erster und zweiter Thiol-Gruppen der ersten und zweiten Peptide gebildet wurde,
jede der ersten und zweiten Peptidketten eine Kette mit zwischen 5 und 25 Aminosäuren aufweist, wobei jede Aminosäure ein D-Enantiomer oder ein L-Enantiomer ist und aus einem Satz von weniger als 6 Aminosäuren ausgewählt wurde,
jede der ersten und zweiten Peptidketten verschiedene Aminosäuresequenzen haben,
das erste Peptid über eine Befestigungsgruppe an einem Substrat immobilisiert ist, wobei die Befestigungsgruppe aus der Gruppe bestehend aus einer hydrophoben Gruppe und einer Gruppe, die eine kovalente Bindung mit dem Substrat bildet, ausgewählt ist und wobei
die ersten und zweiten Peptidketten kombinatorisch derart variiert sind, dass alle möglichen Kombinationen der Aminosäuren, die in dem Satz vorliegen, in dem Array vorliegen und das Array seperate, räumlich adressierbare Stellen aufweist und jede Stelle ein unterschiedliches Einfangreagenz aufweist.

2. Ein Array, wie in Anspruch 1 beansprucht, wobei jede Aminosäure aus einem Satz von 4 Aminosäuren ausgewählt wurde.

3. Eine Array, wie in einem der vorangehenden Ansprüche beantragt, wobei der Ligand eine eukaryotische Zelle, eine prokaryotische Zelle, ein Virus, ein Bakteriophage, ein Prion, eine Spore, ein Pollenkorn, ein Allergen, eine Nukleinsäure, ein Protein, ein Peptid, ein Kohlenhydrat, ein Lipid, eine anorganische Verbindung oder eine organische Verbindung aufweist.

## Revendications

1. Réseau d'agents de capture peptidiques dimériques se liant à un ligand pour capturer un analyte dans un échantillon, dans lequel :
chacun desdits agents de capture se liant à un ligand comprend une première et une seconde chaîne peptidique sous forme de dimère au moyen d'une liaison covalente entre celles-ci, où ladite liaison covalente a été formée par une réaction d'un premier et d'un second groupe thiol sur lesdits premier et second peptides,
chacune desdites première et seconde chaînes peptidiques comprend une chaîne de 5 à 25 acides aminés dans laquelle chaque acide aminé est un énantiomère D ou un énantiomère L, et a été sélectionné parmi un ensemble de moins de 6 acides aminés,
chacune desdites première et seconde chaînes peptidiques possède des séquences d'acides aminés différentes,
ledit premier peptide est immobilisé via un fragment d'attachement à un substrat, ledit fragment d'attachement étant sélectionné dans le groupe consistant en un fragment hydrophobe et un fragment formant une liaison covalente avec le substrat, et dans lequel
la première et la seconde chaîne peptidique varient de manière combinatoire de sorte que toutes les combinaisons possibles des acides aminés présents dans ledit ensemble soient représentées dans le réseau, et ledit réseau comprend des emplacements discrets adressables dans l'espace et chaque emplacement comprend un agent de capture différent.

2. Réseau selon la revendication 1, dans lequel chaque acide aminé a été sélectionné parmi un ensemble de 4 acides aminés.

3. Réseau selon l'une quelconque des revendications précédentes, dans lequel le ligand comprend une cellule eucaryote, une cellule procaryote, un virus, un bactériophage, un prion, une spore, un grain de pollen, un allergène, un acide nucléique, une protéine, un peptide, un glucide, un lipide, un composé inorganique ou un composé organique.


### SEQUENCE LISTING

<110> Sharp Kabushiki Kaisha
<120> NOVEL CAPTURE AGENTS FOR BINDING A LIGAND
<130> 05R00964
<150> GB 0525915.5
   <151> 2005-12-20
<160> 66
<170> PatentIn version 3.3
<210> 1
   <211> 13
   <212> PRT
   <213> Artificial Sequence
<220>
   <222> Synthetic Construct
<220>
   <221> BINDING
   <222> (6)..(6)
   <223> Ligand binding residue
<220>
   <221> BINDING
   <222> (8)..(8)
   <223> Ligand binding residue
<220>
   <221> BINDING
   <222> (10).. (10)
   <223> Ligand binding residue
<400> 1
   Phe Gly Phe Cys Phe Xaa Phe Xaa Phe Xaa Phe Gly Phe
   1 5 10
<210> 2
   <211> 7
   <212> PRT
<213> Artificial Sequence
<220>
   <223> Synthetic Construct
<220>
   <221> SITE
   <222> (1)..(1)
   <223> Cysteine with activated thiol
<220>
   <221> BINDING
   <222> (2)..(2)
   <223> Ligand binding residue
<220>
   <221> BINDING
   <222> (4)..(4)
   <223> Ligand binding residue
<220>
   <221> BINDING
   <222> (6)..(6)
   <223> Ligand binding residue
<400> 2
   Xaa Xaa Phe Xaa Phe Xaa Phe
   1 5
<210> 3
   <211> 9
   <212> PRT
   <213> Artificial sequence
<220>
   <223> Synthetic Construct
<220>
   <221> SITE
   <222> (1)..(1)
   <223> Norleucine
<220>
   <221> SITE
   <222> (3)..(3)
   <223> Norleucine
<220>
   <221> SITE
   <222> (5)..(5)
   <223> Norleucine
<220>
   <221> SITE
   <222> (7)..(7)
   <223> Norleucine
<220>
   <221> SITE
   <222> (9)..(9)
   <223> Norleucine
<400> 3
   Xaa Asp Xaa Ala Xaa Ser Xaa Lys Xaa
   1 5
<210> 4
   <211> 9
   <212> PRT
   <213> Artificial sequence
<220>
   <223> Synthetic Construct
<400> 4
   Phe Asp Phe Ala Phe Ser Phe Lys Phe
   1 5
<210> 5
   <211> 9
   <212> PRT
   <213> Artificial sequence
<220>
   <223> Synthetic Construct
<400> 5
   Ser Asp Ser Ala Ser Ser Ser Lys Ser
   1 5
<210> 6
   <211> 9
   <212> PRT
   <213> Artificial sequence
<220>
   <223> Synthetic Construct
<400> 6
   Asp Asp Asp Ala Asp Ser Asp Lys Asp
   1 5
<210> 7
   <211> 7
   <212> PRT
   <213> Artificial sequence
<220>
   <223> Synthetic Construct
<220>
   <221> SITE
   <222> (2)..(2)
   <223> Norleucine
<220>
   <221> SITE
   <222> (4)..(4)
   <223> Norleucine
<220>
   <221> SITE
   <222> (6)..(6)
   <223> Norleucine
<400> 7
   Asp Xaa Ala Xaa Ser Xaa Lys
   1 5
<210> 8
   <211> 7
   <212> PRT
   <213> Artificial sequence
<220>
   <223> Synthetic Construct
<400> 8
   Asp Phe Ala Phe Ser Phe Lys
   1 5
<210> 9
   <211> 7
   <212> PRT
   <213> Artificial sequence
<220>
   <223> Synthetic Construct
<400> 9
   Asp Ser Ala Ser Ser Ser Lys
   1 5
<210> 10
   <211> 7
   <212> PRT
   <213> Artificial sequence
<220>
   <223> Synthetic Construct
<400> 10
   Asp Asp Ala Asp Ser Asp Lys
   1 5
<210> 11
   <211> 9
   <212> PRT
   <213> Artificial sequence
<220>
   <223> Synthetic Construct
<220>
   <221> SITE ,
   <222> (1)..(1)
   <223> Norleucine
<220>
   <221> SITE
   <222> (3)..(3)
   <223> Norleucine
<220>
   <221> SITE
   <222> (5)..(5)
   <223> Norleucine
<220>
   <221> SITE
   <222> (6)..(6)
   <223> Norleucine
<220>
   <221> misc_feature
   <222> (7)..(7)
   <223> Xaa can be any naturally occurring amino acid
<220>
   <221> SITE
   <222> (9)..(9)
   <223> Norleucine
<400> 11
   Xaa Asp Xaa Asp Xaa Asp Xaa Asp Xaa
   1 5
<210> 12
   <211> 9
   <212> PRT
   <213> Artificial sequence
<220>
   <223> Synthetic Construct
<400> 12
   Phe Asp Phe Asp Phe Asp Phe Asp Phe
   1 5
<210> 13
   <211> 9
   <212> PRT
   <213> Artificial sequence
<220>
   <223> Synthetic Construct
<400> 13
   Ser Asp Ser Asp Ser Asp Ser Asp Ser
   1 5
<210> 14
   <211> 9
   <212> PRT
   <213> Artificial sequence
<220>
   <223> Synthetic Construct
<400> 14
   Asp Asp Asp Asp Asp Asp Asp Asp Asp
   1 5
<210> 15 <210> 15

   <211> 7
   <212> PRT
   <213> Artificial sequence
<220>
   <223> Synthetic Construct
<220>
   <221> SITE
   <222> (2).. (2)
   <223> Norleucine
<220>
   <221> SITE
   <222> (4)..(4)
   <223> Norleucine
<220>
   <221> SITE
   <222> (6)..(6)
   <223> Norleucine
<400> 15
   Asp Xaa Asp Xaa Asp Xaa Asp
   1 5
<210> 16
   <211> 7
   <212> PRT
   <213> Artificial sequence
<220>
   <223> Synthetic Construct
<400> 16
   Asp Phe Asp Phe Asp Phe Asp
   1 5
<210> 17
   <211> 7
   <212> PRT
   <213> Artificial sequence
<220>
   <223> Synthetic Construct
<400> 17
   Asp Ser Asp Ser Asp Ser Asp
   1 5
<210> 18
   <211> 7
   <212> PRT
   <213> Artificial sequence
   <220>
   <223> Synthetic Construct
<400> 18
   Asp Asp Asp Asp Asp Asp Asp
   1 5
<210> 19
   <211> 11
   <212> PRT
   <213> Artificial sequence
<220>
   <223> Synthetic Construct
<400> 19
   Phe Gly Phe Ser Phe Ala Phe Asp Phe Gly Phe
   1 5 10
<210> 20
   <211> 13
   <212> PRT
   <213> Artificial sequence
<220>
   <223> Synthetic Construct
<400> 20
   Phe Gly Phe Gly Phe Ser Phe Ala Phe Asp Phe Gly Phe
   1 5 10
<210> 21
   <211> 15
   <212> PRT
   <213> Artificial sequence
<220>
   <223> Synthetic Construct
<400> 21
   Phe Gly Phe Gly Phe Gly Phe Ser Phe Ala Phe Asp Phe Gly Phe
   1 5 10 15
<210> 22
   <211> 17
   <212> PRT
   <213> Artificial sequence
<220>
   <223> Synthetic Construct
<400> 22
   Phe Gly Phe Gly Phe Gly Phe Gly Phe Ser Phe Ala Phe Asp Phe Gly
   1 5 10 15
   Phe
<210> 23
   <211> 19
   <212> PRT
   <213> Artificial sequence
<220>
   <223> Synthetic Construct
<400> 23
   Phe Gly Phe Gly Phe Gly Phe Gly Phe Gly Phe Ser Phe Ala Phe Asp
   1 5 10 15
   Phe Gly Phe
<210> 24
   <211> 7
   <212> PRT
   <213> Artificial sequence
<220>
   <223> Synthetic Construct
<400> 24
   Gly Ser Phe Ala Phe Asp Phe
   1 5
<210> 25
   <211> 7
   <212> PRT
   <213> Artificial sequence
<220>
   <223> Synthetic Construct
<400> 25
   Gly Ser Gly Ala Phe Asp Phe
   1 5
<210> 26
   <211> 13
   <212> PRT
   <213> Artificial sequence
<220>
   <223> Synthetic Construct
<400> 26
   Phe Gly Phe Lys Phe Gly Phe Asp Phe Gly Phe Ala Phe
   1               5                   10
<210> 27
   <211> 13
   <212> PRT
   <213> Artificial sequence
<220>
   <223> Synthetic Construct
<400> 27
   Phe Gly Phe Lys Phe Gly Phe Asp Phe Gly Phe Ser Phe
   1               5                   10
<210> 28
   <211> 13
   <212> PRT
   <213> Artificial sequence
<220>
   <223> Synthetic Construct
<400> 28
   Phe Gly Phe Lys Phe Gly Phe Asp Phe Gly Phe Cys Phe
   1               5                   10
<210> 29
   <211> 13
   <212> PRT
   <213> Artificial sequence
<220>
   <223> Synthetic Construct
<220>
   <221> SITE
   <222> (12)..(12)
   <223> nitropyridylthio activated cysteiyl
<400> 29
   Phe Gly Phe Lys Phe Gly Phe Asp Phe Gly Phe Xaa Phe
   1 5 10
<210> 30
   <211> 19
   <212> PRT
   <213> Artificial sequence
<220>
   <223> Synthetic Construct
<400> 30
   Phe Gly Phe Gly Phe Gly Phe Gly Phe Gly Phe Ser Phe Ala Phe Asp
   1 5 10 15
   Phe Gly Phe
<210> 31
   <211> 23
   <212> PRT
   <213> Artificial sequence
<220>
   <223> Synthetic Construct
<220>
   <221> SITE
   <222> (18)..(18)
   <223> diaminobutyryl residue
<220>
   <221> SITE
   <222> (20)..(20)
   <223> diaminobutyryl residue
<400> 31
   Phe Gly Phe Gly Phe Gly Phe Gly Phe Gly Phe Gly Phe Gly Phe Cys
   1 5 10 15
   Phe Xaa Phe Xaa Phe Gly Phe
   20
<210> 32
   <211> 23
   <212> PRT
   <213> Artificial sequence
<220>
   <223> Synthetic Construct
<220>
   <221> SITE
   <222> (18)..(18)
   <223> diaminobutyryl residue
<220>
   <221> SITE
   <222> (20)..(20)
   <223> homoseryl residue
<400> 32
   Phe Gly Phe Gly Phe Gly Phe Gly Phe Gly Phe Gly Phe Gly Phe Cys
   1               5                   10                  15
   Phe Xaa Phe Xaa Phe Gly Phe
               20
<210> 33
   <211> 23
   <212> PRT
   <213> Artificial sequence
<220>
   <223> Synthetic Construct
<220>
   <221> SITE
   <222> (18)..(18)
   <223> diaminobutyryl residue
<220>
   <221> SITE
   <222> (20)..(20)
   <223> aminobutyryl residue
<400> 33
   Phe Gly Phe Gly Phe Gly Phe Gly Phe Gly Phe Gly Phe Gly Phe Cys
   1               5                   10                  15
   Phe Xaa Phe Xaa Phe Gly Phe
               20
<210> 34
   <211> 23
   <212> PRT
   <213> Artificial sequence
<220>
   <223> Synthetic Construct
<220>
   <221> SITE
   <222> (18)..(18)
   <223> diaminobutyryl residue
<400> 34
   Phe Gly Phe Gly Phe Gly Phe Gly Phe Gly Phe Gly Phe Gly Phe Cys
   1 5 10 15
   Phe Xaa Phe Asp Phe Gly Phe
   20
<210> 35
   <211> 23
   <212> PRT
   <213> Artificial sequence
<220>
   <223> Synthetic Construct
<220>
   <221> SITE
   <222> (18)..(18)
   <223> homoseryl residue
<220>
   <221> SITE
   <222> (20)..(20)
   <223> diaminobutyryl residue
<400> 35
   Phe Gly Phe Gly Phe Gly Phe Gly Phe Gly Phe Gly Phe Gly Phe Cys
   1 5 10 15
   Phe Xaa Phe Xaa Phe Gly Phe
   20
<210> 36
   <211> 23
   <212> PRT
   <213> Artificial sequence
<220>
   <223> Synthetic Construct
<220>
   <221> SITE
   <222> (18)..(18)
   <223> homoseryl residue
<220>
   <221> SITE
   <222> (20)..(20)
   <223> homoseryl residue
<400> 36
   Phe Gly Phe Gly Phe Gly Phe Gly Phe Gly Phe Gly Phe Gly Phe Cys
   1               5                   10                  15
   Phe Xaa Phe Xaa Phe Gly Phe
               20
<210> 37
   <211> 23
   <212> PRT
   <213> Artificial sequence
<220>
   <223> Synthetic Construct
<220>
   <221> SITE
   <222> (18)..(18)
   <223> homoseryl residue
<220>
   <221> SITE
   <222> (20)..(20)
   <223> aminobutyryl residue
<400> 37
   Phe Gly Phe Gly Phe Gly Phe Gly Phe Gly Phe Gly Phe Gly Phe Cys
   1               5                   10                  15
   Phe Xaa Phe Xaa Phe Gly Phe
               20
<210> 38
   <211> 23
   <212> PRT
   <213> Artificial sequence
<220>
   <223> Synthetic Construct
<220>
   <221> SITE
   <222> (18)..(18)
   <223> homoseryl residue
<400> 38
   Phe Gly Phe Gly Phe Gly Phe Gly Phe Gly Phe Gly Phe Gly Phe Cys
   1 5 10 15
   Phe Xaa Phe Asp Phe Gly Phe
   20
<210> 39
   <211> 23
   <212> PRT
   <213> Artificial sequence
<220>
   <223> Synthetic Construct
<220>
   <221> SITE
   <222> (18)..(18)
   <223> aminobutyryl residue
<220>
   <221> SITE
   <222> (20)..(20)
   <223> diaminobutyryl residue
<400> 39
   Phe Gly Phe Gly Phe Gly Phe Gly Phe Gly Phe Gly Phe Gly Phe Cys
   1 5 10 15
   Phe Xaa Phe Xaa Phe Gly Phe
   20
<210> 40
   <211> 23
   <212> PRT
   <213> Artificial sequences
<220>
   <223> Synthetic Construct
<220>
   <221> SITE
   <222> (18)..(18)
   <223> Aminobutyryl residue
<220>
   <221> SITE
   <222> (20).. (20)
   <223> homoseryl residue
<400> 40
   Phe Gly Phe Gly Phe Gly Phe Gly Phe Gly Phe Gly Phe Gly Phe Cys
   1 5 10 15
   Phe Xaa Phe Xaa Phe Gly Phe
   20
<210> 41
   <211> 23
   <212> PRT
   <213> Artificial sequence
   <220>
   <223> Synthetic Construct
<220>
   <221> SITE
   <222> (18)..(18)
   <223> aminobutyryl residue
<220>
   <221> SITE
   <222> (20)..(20)
   <223> aminobutyryl residue
<400> 41
   Phe Gly Phe Gly Phe Gly Phe Gly Phe Gly Phe Gly Phe Gly Phe Cys
   1 5 10 15
   Phe Xaa Phe Xaa Phe Gly Phe
   20
<210> 42
   <211> 23
   <212> PRT
   <213> Artificial sequence
<220>
   <223> Synthetic Construct
<220>
   <221> SITE
   <222> (18)..(18)
   <223> aminobutyryl residue
<400> 42
   Phe Gly Phe Gly Phe Gly Phe Gly Phe Gly Phe Gly Phe Gly Phe Cys
   1 5 10 15
   Phe Xaa Phe Asp Phe Gly Phe
   20
<210> 43
   <211> 23
   <212> PRT
   <213> Artificial sequence
<220>
   <223> Synthetic Construct
<220>
   <221> SITE
   <222> (20)..(20)
   <223> diaminobutyryl residue
<400> 43
   Phe Gly Phe Gly Phe Gly Phe Gly Phe Gly Phe Gly Phe Gly Phe Cys
   1 5 10 15
   Phe Asp Phe Xaa Phe Gly Phe
   20
<210> 44
   <211> 23
   <212> PRT
   <213> Artificial sequence
<220>
   <223> Synthetic Construct
<220>
   <221> SITE
   <222> (20)..(20)
   <223> homoseryl residue
<400> 44
   Phe Gly Phe Gly Phe Gly Phe Gly Phe Gly Phe Gly Phe Gly Phe Cys
   1 5 10 15
   Phe Asp Phe Xaa Phe Gly Phe
   20
<210> 45
   <211> 23
   <212> PRT
   <213> Artificial sequence
<220>
   <223> Synthetic Construct
<220>
   <221> SITE
   <222> (20)..(20)
   <223> aminobutyryl residue
<400> 45
   Phe Gly Phe Gly Phe Gly Phe Gly Phe Gly Phe Gly Phe Gly Phe Cys
   1               5                   10                  15
   Phe Asp Phe Xaa Phe Gly Phe
               20
<210> 46
   <211> 23
   <212> PRT
   <213> Artificial sequence
<220>
   <223> Synthetic Construct
<400> 46
   Phe Gly Phe Gly Phe Gly Phe Gly Phe Gly Phe Gly Phe Gly Phe Cys
   1               5                   10                  15
   Phe Asp Phe Asp Phe Gly Phe
               20
<210> 47
   <211> 7
   <212> PRT
   <213> Artificial sequence
<220>
   <223> Synthetic Construct
<220>
   <221> SITE
   <222> (1)..(1)
   <223> nitropyridylthio activated cysteiyl
<220>
   <221> SITE
   <222> (2)..(2)
   <223> diaminobutyryl residue
<220>
   <221> SITE
   <222> (4)..(4)
   <223> diaminobutyryl residue
<400> 47
   Xaa Xaa Phe Xaa Phe Gly Phe
   1               5
<210> 48
   <211> 7
   <212> PRT
   <213> Artificial sequence
<220>
   <223> Synthetic Construct
<220>
   <221> SITE
   <222> (1)..(1)
   <223> nitropyridylthio activated cysteiyl
<220>
   <221> SITE
   <222> (2)..(2)
   <223> diaminobutyryl residue
<220>
   <221> SITE
   <222> (4)..(4)
   <223> homoseryl residue
<400> 48
   Xaa Xaa Phe Xaa Phe Gly Phe
   1               5
<210> 49
   <211> 7
   <212> PRT
   <213> Artificial sequence
<220>
   <223> Synthetic Construct
<220>
   <221> SITE
   <222> (1)..(1)
   <223> nitropyridylthio activated cysteiyl
<220>
   <221> SITE
   <222> (2)..(2)
   <223> diaminobutyryl desidue
<220>
   <221> SITE
   <222> (4)..(4)
   <223> aminobutyryl desidue
<400> 49
   Xaa Xaa Phe Xaa Phe Gly Phe
   1 5
<210> 50
   <211> 7
   <212> PRT
   <213> Artificial sequence
<220>
   <223> Synthetic Construct
<220>
   <221> SITE
   <222> (1)..(1)
   <223> nitropyridylthio activated cysteiyl
<220>
   <221> SITE
   <222> (2)..(2)
   <223> diaminobutyryl residue
<400> 50
   Xaa Xaa Phe Asp Phe Gly Phe
   1 5
<210> 51
   <211> 7
   <212> PRT
   <213> Artificial sequence
<220>
   <223> Synthetic Construct
<220>
   <221> SITE
   <222> (1)..(1)
   <223> nitropyridylthio activated cysteiyl
<220>
   <221> SITE
   <222> (2)..(2)
   <223> homoseryl residue
<220>
   <221> SITE
   <222> (4)..(4)
   <223> diaminobutyryl residue
<400> 51
   Xaa Xaa Phe Xaa Phe Gly Phe
   1 5
<210> 52
   <211> 7
   <212> PRT
   <213> Artificial sequence
<220>
   <223> Synthetic Construct
<220>
   <221> SITE
   <222> (1)..(1)
   <223> nitropyridylthio activated cysteiyl
<220>
   <221> SITE
   <222> (2)..(2)
   <223> homoseryl residue
<220>
   <221> SITE
   <222> (4)..(4)
   <223> homoseryl residue
<400> 52
   Xaa Xaa Phe Xaa Phe Gly Phe
   1 5
<210> 53
   <211> 7
   <212> PRT
   <213> Artificial sequence
<220>
   <223> Synthetic Construct
<220>
   <221> SITE
   <222> (1)..(1)
   <223> nitropyridylthio activated cysteiyl
<220>
   <221> SITE
   <222> (2)..(2)
   <223> homoseryl residue
<220>
   <221> SITE
   <222> (4)..(4)
   <223> aminobutyryl residue
<400> 53
   Xaa Xaa Phe Xaa Phe Gly Phe
   1               5
<210> 54
   <211> 7
   <212> PRT
   <213> Artificial sequence
<220>
   <223> Synthetic Construct
<220>
   <221> SITE
   <222> (1)..(1)
   <223> nitropyridylthio activated cysteiyl
<220>
   <221> SITE
   <222> (2)..(2)
   <223> homoseryl residue
<400> 54
   Xaa Xaa Phe Asp Phe Gly Phe
   1               5
<210> 55
   <211> 7
   <212> PRT
   <213> Artificial sequence
<220>
   <223> Synthetic Construct
<220>
   <221> SITE
   <222> (1)..(1)
   <223> nitropyridylthio activated cysteiyl
<220>
   <221> SITE
   <222> (2)..(2)
   <223> aminobutyryl residue
<220>
   <221> SITE
   <222> (4)..(4)
   <223> diaminobutyryl residue
<400> 55
   Xaa Xaa Phe Xaa Phe Gly Phe
   1 5
<210> 56
   <211> 7
   <212> PRT
   <213> Artificial sequence
<220>
   <223> Synthetic Construct
<220>
   <221> SITE
   <222> (1)..(1)
   <223> nitropyridylthio activated cysteiyl
<220>
   <221> SITE
   <222> (2)..(2)
   <223> aminobutyryl residue
<220>
   <221> SITE
   <222> (4)..(4)
   <223> homoseryl residue
<400> 56
   Xaa Xaa Phe Xaa Phe Gly Phe
   1 5
<210> 57
   <211> 7
   <212> PRT
   <213> Artificial sequence
<220>
   <223> Synthetic Construct
<220>
   <221> SITE
   <222> (1)..(1)
   <223> nitropyridylthio activated cysteiyl
<220>
   <221> SITE
   <222> (2)..(2)
   <223> aminobutyryl residue
<220>
   <221> SITE
   <222> (4)..(4)
   <223> aminobutyryl residue
<400> 57
   Xaa Xaa Phe Xaa Phe Gly Phe
   1 5
<210> 58
   <211> 7
   <212> PRT
   <213> Artificial sequence
<220>
   <223> Synthetic Construct
<220>
   <221> SITE
   <222> (1)..(1)
   <223> nitropyridylthio activated cysteiyl
<220>
   <221> SITE
   <222> (2)..(2)
   <223> aminobutyryl residue
<400> 58
   Xaa Xaa Phe Asp Phe Gly Phe
   1 5
<210> 59
   <211> 7
   <212> PRT
   <213> Artificial sequence
<220>
   <223> Synthetic Construct
<220>
   <221> SITE
   <222> (1)..(1)
   <223> nitropyridylthio activated cysteiyl
<220>
   <221> SITE
   <222> (4)..(4)
   <223> diaminobutyryl residue
<400> 59
   Xaa Asp Phe Xaa Phe Gly Phe
   1 5
<210> 60
   <211> 7
   <212> PRT
   <213> Artificial sequence
<220>
   <223> Synthetic Construct
<220>
   <221> SITE
   <222> (1)..(1)
   <223> nitropyridylthio activated cysteiyl
<220>
   <221> SITE
   <222> (4)..(4)
   <223> homoseryl residue
<400> 60
   Xaa Asp Phe Xaa Phe Gly Phe
   1 5
<210> 61
   <211> 7
   <212> PRT
   <213> Artificial sequence
<220>
   <223> Synthetic Construct
<220>
   <221> SITE
   <222> (1)..(1)
   <223> nitropyridylthio activated cysteiyl
<220>
   <221> SITE
   <222> (4)..(4)
   <223> aminobutyryl residue.
<400> 61
   Xaa Asp Phe Xaa Phe Gly Phe
   1 5
<210> 62
   <211> 7
   <212> PRT
   <213> Artificial sequence
<220>
   <223> Synthetic Construct
<220>
   <221> SITE
   <222> (1)..(1)
   <223> nitropyridylthio activated cysteiyl
<400> 62
   Xaa Asp Phe Asp Phe Gly Phe
   1 5
<210> 63
   <211> 7
   <212> PRT
   <213> Artificial sequence
<220>
   <223> Synthetic Construct
<220>
   <221> SITE
   <222> (1)..(1)
   <223> nitropyridylthio activated cysteiyl
<220>
   <221> SITE
   <222> (2)..(2)
   <223> diaminobutyryl residue
<220>
   <221> SITE
   <222> (4)..(4)
   <223> diaminobutyryl residue
<400> 63
   Xaa Xaa Phe Xaa Phe Gly Phe
   1 5
<210> 64
   <211> 7
   <212> PRT
   <213> Artificial sequence
<220>
   <223> Synthetic Construct
<226>
   <221> SITE
   <222> (1)..(1)
   <223> nitropyridylthio activated cysteiyl
<220>
   <221> SITE
   <222> (2)..(2)
   <223> homoseryl residue
<220>
   <221> SITE
   <222> (4)..(4)
   <223> homoseryl residue
<400> 64
   Xaa Xaa Phe Xaa Phe Gly Phe
   1 5
<210> 65
   <211> 7
   <212> PRT
   <213> Artificial sequence
<220>
   <223> Synthetic Construct
<220>
   <221> SITE
   <222> (1)..(1)
   <223> nitropyridylthio activated cysteiyl
<220>
   <221> SITE
   <222> (2)..(2)
   <223> aminobutyryl residue
<220>
   <221> SITE
   <222> (4)..(4)
   <223> aminobutyryl residue
<400> 65
   Xaa Xaa Phe Xaa Phe Gly Phe
   1 5
<210> 66
   <211> 7
   <212> PRT
   <213> Artificial sequence
<220>
   <223> Synthetic Construct
<220>
   <221> SITE
   <222> (1)..(1)
   <223> nitropyridylthio activated cysteiyl
<400> 66
   Xaa Asp Phe Asp Phe Gly Phe
   1 5